# EUROPEAN PATENT APPLICATION

(11) **EP 4 822 017 A1**
(43) Date of publication of application: **14.10.2026**
(21) Application number: 24900622.2
(22) Date of filing: 03.12.2024
(51) Int. Cl.: C12N 5/10, C12M 1/00, C12N 5/0789, C12N 15/11

(54) **METHOD FOR PRODUCING INDUCED PLURIPOTENT STEM CELLS**

(30) Priority: 04.12.2023 JP 2023204958
(71) Applicant: CiRA Foundation, Kyoto-shi, Kyoto 606-8397 (JP)
(72) Inventor: SHIMIZU, Eiko, Kyoto-shi, Kyoto 606-8397 (JP)
(74) Representative: Regimbeau
(86) International application number: PCT/JP2024/042748
(87) International publication number: WO 2025/121328

(57) **Abstract**

The present invention provides a method for producing iPS cells using a cell processing device, the cell processing device including a closed system portion in which a sealed material supply container and a sealed chamber rotatable for centrifugation are connected via a connecting conduit, the production method comprising: a step of separating blood cells from whole blood by centrifugation; a step of bringing the blood cells into contact with a reprogramming factor; and a step of establishing iPS cells from the blood cells, which are performed in the stated order in the sealed rotatable chamber while maintaining closedness of the closed system portion.

## Description

### TECHNICAL FIELD

The present invention relates to a method for producing induced pluripotent stem cells using a cell processing device, and a method for producing differentiated cells using the production method.

### BACKGROUND ART

In recent years, research into regenerative medicine using differentiated cells derived from induced pluripotent stem cells (hereinafter also referred to as iPS cells) is currently being actively conducted. In particular, a treatment by which iPS cells are established from a patient's somatic cells (e.g., peripheral blood mononuclear cells or the like) and then various differentiated cells or organoids induced to differentiate from the iPS cells are transplanted into the patient (autotransplantation) has attracted attention as a treatment that can reduce the risk of rejection (Non-Patent Literature 1 and 2). Patent Literature 1 (Japanese Unexamined Patent Publication No. 2017-195905) discloses a system capable of producing stem cells. Also, CAR-T therapy and other treatments using a patient's own somatic cells are beginning to be applied clinically, and Patent Literature 2 (Japanese Unexamined Patent Publication No. 2022-8735) discloses a cell production system and a cell production method, with which costs can be reduced by reducing the number of steps in a treatment method using CAR-T cells.

### Citation List

### Patent Literature

Patent Literature 1: Japanese Unexamined Patent Publication No. 2017-195905
Patent Literature 2: Japanese Unexamined Patent Publication No. 2022-8735
Patent Literature 3: WO2020/040135
Patent Literature 4: Japanese Unexamined Patent Publication No. 2020-182488
Patent Literature 5: Japanese Unexamined Patent Publication No. 2014-018185
Patent Literature 6: Japanese Unexamined Patent Publication No. 2011-505890

### Non-Patent Literature

Non-Patent Literature 1: Shinsuke Yoshida., et al., CLINICAL AND TRANSLATIONAL RESOURCE AND TECHNOLOGY INSIGHTS VOLUME 4, ISSUE 1, P51-66.E10, JANUARY 13, 2023
Non-Patent Literature 2: Madrid, M., et al., Current Protocols, 1, e88. doi: 10.1002/cpzl.88

### SUMMARY OF INVENTION

### Technical Problem

One of the features of iPS cells is that they can be produced from a patient's own somatic cells, making them an effective means of minimizing the risk of rejection. However, establishment of iPS cells from somatic cells requires multiple and long processing steps and involves high costs for many dedicated devices and persons who operate and manage them.

If multiple steps of isolating somatic cells from the peripheral blood collected from the patient, establishing iPS cells, carrying out their expansion culture, and then forming differentiated cells can be automatically performed in a single closed-type device, the cost for producing autologous iPS cells can be much smaller than in conventional production. However, there has been no conventional cell processing device optimized to perform all the above-mentioned multiple steps automatically and continuously, and there has also been no attempt to allow an existing cell processing device to perform the multiple steps.

For example, Patent Literature 3 (WO2020/040135) discloses a step of establishing iPS cells from somatic cells separated from whole blood, but a step of separating somatic cells from whole blood and the step of establishing iPS cells are performed in mutually different containers.

Patent Literature 4 (Japanese Unexamined Patent Publication No. 2020-182488) discloses transduction in a centrifugation chamber provided in a cell processing device, but does not disclose that multiple steps from separation of somatic cells from whole blood to establishment of iPS cells from the somatic cells are performed in a single cell processing device. Also, the Patent Literature 4 does not disclose adherent culture useful for culturing iPS cells which are adherent cells.

Patent Literature 5 (Japanese Unexamined Patent Publication No. 2014-018185) discloses adherent culture of iPS cells in a closed system, but does not disclose that somatic cells are separated from whole blood and iPS cells are established from the somatic cells in an identical container within an identical closed system.

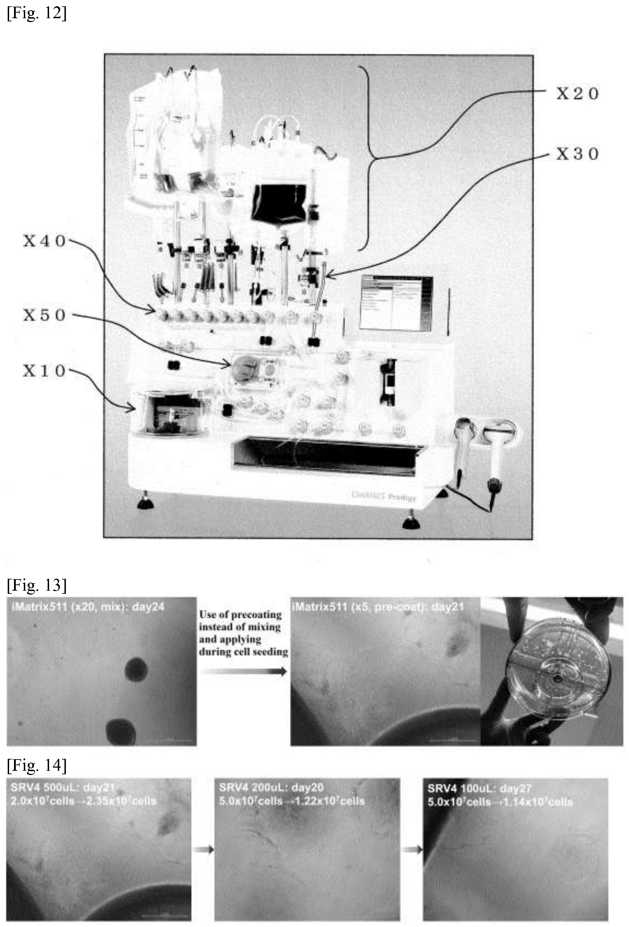

On the other hand, cell processing devices configured to automatically perform sequential processing on cells while maintaining the closed system have been proposed by device manufacturers (e.g., CliniMACS Prodigy (registered trademark) manufactured by Miltenyi Biotec). FIG. 12 shows a photograph showing an example of a state where such a cell processing device is used. The cell processing device illustrated in FIG. 12 includes a closed system portion for processing cells, in which a sealed container (material supply bag) X20 storing cell processing materials and a sealed chamber X10 rotatable for centrifugation are connected via a connecting conduit (soft tube) X30. The connecting conduit X30 passes through many pinch valves X40 that can be controlled to open and close, and one peristaltic pump X50, thereby enabling control of communication and blocking of the conduit and control of feeding of the materials while maintaining the closed system. Also, the sealed chamber X10 is configured to be rotatable for centrifugation in a state where its connection to the connecting conduit is maintained.

However, for conventional cell processing using a cell processing device as shown in FIG. 12 (in particular, CliniMACS Prodigy), a procedure to continuously perform the above-mentioned multiple steps necessary for producing iPS cells has not yet been disclosed. Moreover, the centrifugation function of a sealed chamber in a conventional cell processing device is intended for use in separation at the end of the processing steps to harvest the processed cells. Furthermore, the material that constitutes the sealed chamber in CliniMACS Prodigy is a polycarbonate resin, in order to increase the mechanical strength for the centrifugation function and to make the wall more transparent for observation of the inside, for example. However, due to the nature of the polycarbonate resin, the inner wall surface of the sealed chamber is non-cell-adhesive and is not suitable for effective establishment (adherent culture utilizing the inner wall surface) of iPS cells which are adherent cells.

It is an object of the present invention to provide a production method in which iPS cells are established from whole blood using a conventional cell processing device as described above, in particular, a production method in which iPS cells are established from whole blood in one sealed container (sealed chamber for centrifugation) within one closed system.

### Solution to Problem

Main configurations of the present invention are as follows.
[1] A method for producing induced pluripotent stem cells using a cell processing device,
   the cell processing device including a closed system portion in which a sealed material supply container and a sealed chamber rotatable for centrifugation are connected via a connecting conduit,
   the production method comprising:
      a step (s1) of separating blood cells from whole blood by centrifugation;
      a step (s2) of bringing the blood cells into contact with a reprogramming factor; and
      a step (s3) of establishing induced pluripotent stem cells from the blood cells, which are performed in the stated order in the sealed rotatable chamber while maintaining closedness of the closed system portion.
[2] The method for producing induced pluripotent stem cells according to [1],
   wherein the sealed chamber includes an inlet port and an outlet port and is configured to perform material inflow and outflow via the inlet port and the outlet port in a rotated state for centrifugation or in a state where the rotation is stopped, and
   in the step (s1), a fraction containing the blood cells separated by centrifugation remains in the sealed chamber, and another fraction is discharged from the sealed chamber.
[3] The method for producing induced pluripotent stem cells according to [2],
   wherein the cell processing device further includes, as a part of a configuration of the closed system portion, a magnetic separation column connected to the sealed chamber via the connecting conduit, and
   in the step (s1),
      after the centrifugation, the fraction containing the blood cells remains in the sealed chamber,
      lymphoid cells among the blood cells are further labeled with magnetic beads, sent to the magnetic separation column, and magnetically separated from the other blood cells, and
      the lymphoid cells labeled with the magnetic beads are returned to the sealed chamber.
[4] The method for producing induced pluripotent stem cells according to any one of [1] to [3], wherein the step (s2) is performed in one of a state where the sealed chamber is rotated and a state where the sealed chamber stands still, or in a combination of these states.
[5] The method for producing induced pluripotent stem cells according to any one of [1] to [4],
   wherein an internal space of the sealed chamber as defined by a side wall surface surrounding the internal space has a cylindrical shape, a geometrical rotation center line of the cylindrical shape corresponds to a center axis line of rotation movement in centrifugation in the sealed chamber,
   the cylindrical shape has a bottom surface area of 90 to 200 cm², and
   a relative centrifugal force acting on a content in the sealed chamber in the centrifugation is 100 to 500 G.
[6] The method for producing induced pluripotent stem cells according to any one of [1] to [5], wherein the sealed chamber has a volume of 300 to 700 ml.
[7] The method for producing induced pluripotent stem cells according to any one of [1] to [6], wherein the centrifugation in the step (s1) is density gradient centrifugation performed by adding a density gradient centrifugation medium into the sealed chamber.
[8] The method for producing induced pluripotent stem cells according to any one of [1] to [7], wherein at least an inner wall surface of the sealed chamber includes a polycarbonate resin portion.
[9] The method for producing induced pluripotent stem cells according to any one of [1] to [8], wherein the steps (s2) and (s3) are performed in the presence of a scaffold material.
[10] The method for producing induced pluripotent stem cells according to [9], wherein the step (s1) is performed in the presence of the scaffold material.
[11] The method for producing induced pluripotent stem cells according to [9] or [10], wherein the scaffold material is adhered to the inner wall surface of the sealed chamber.
[12] The method for producing induced pluripotent stem cells according to any one of [9] to [11], wherein the scaffold material contains a laminin fragment, a cell-adhesive peptide and a polyvinyl acetal resin, or a cell-adhesive peptide and a poly(meth)acrylate resin.
[13] The method for producing induced pluripotent stem cells according to [12], wherein the cell-adhesive peptide contains Arg-Gly-Asp.
[14] The method for producing induced pluripotent stem cells according to any one of [1] to [13], further comprising, after the step (s3), a step (s4) of carrying out expansion culture of the induced pluripotent stem cells in the sealed chamber while maintaining closedness of the closed system portion.
[15] A method for producing differentiated cells,
   the production method comprising:
   the steps (s1) to (s3) in the method for producing induced pluripotent stem cells according to any one of [1] to [13], and
   further comprising, after the step (s3), a step (s5) of inducing differentiation of the induced pluripotent stem cells present in the sealed chamber while maintaining closedness of the closed system portion,
   wherein, in the step (s5), a material necessary for the differentiation induction is supplied into the sealed chamber, and the step (s5) is performed in the sealed chamber.
[16] The method according to [15], further comprising, between the steps (s3) and (s5), a step (s4) of carrying out expansion culture of the induced pluripotent stem cells in the sealed chamber while maintaining closedness of the closed system portion.

### Advantageous Effects of Invention

In the production method according to the present invention, a closed system cell processing device including a sealed chamber with a centrifugation function is used as a container for processing cells. In the production method according to the present invention, the centrifugation function is utilized at the start of all the processing steps to separate blood cells as raw materials for iPS cells from whole blood by centrifugation, and then the blood cells are reprogrammed in the same sealed chamber to establish iPS cells. Furthermore, expansion culture of the iPS cells and formation of differentiated cells are performed in the same sealed chamber. Accordingly, multiple steps from separation of blood cells from whole blood to establishment of iPS cells (and furthermore to formation of differentiated cells) can be continuously performed using one closed system cell processing device. This makes it possible to reduce cell contamination during production, to perform iPS cell production by fewer persons than in conventional production at CPCs by automating production using the device, and to eliminate the need to upgrade the facility to Grade A due to the device being a closed system device, resulting in reduced cost for producing iPS cells.

### Brief Description of Drawings

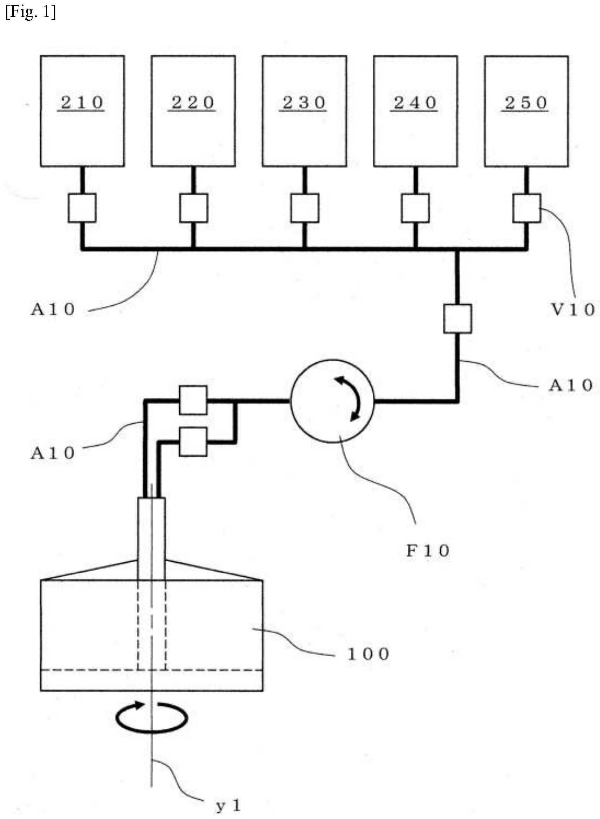

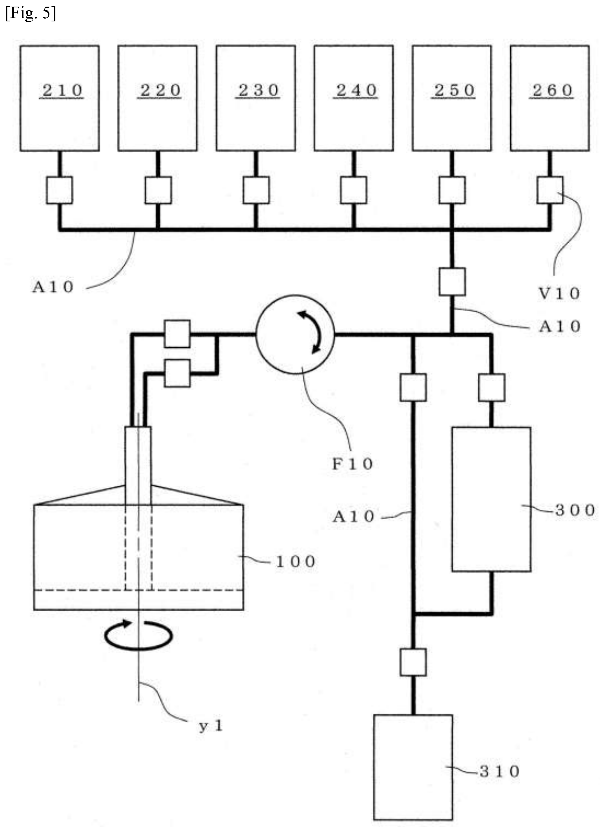

[FIG. 1] FIG. 1 shows a block diagram showing a configuration example of a closed system portion of a cell processing device used in a production method according to the present invention. Each thick black line, indicated by reference numeral A10, indicates a connecting conduit such as a soft tube (this also applies to FIG. 5). Pinch valves that open and close the connecting conduits and a peristaltic pump that feeds materials are additionally depicted on the connecting conduits to clearly show movement and resting of materials in the closed system portion. The pinch valves and the peristaltic pump are located outside the connecting conduits, do not belong to the closed system portion, and belong to a mechanism portion of the cell processing device (this also applies to FIG. 5).
[FIG. 2] FIG. 2 shows a schematic diagram showing a manner of centrifugation in a sealed chamber of a cell processing device used in a production method according to the present invention, showing the inside of the sealed chamber.
[FIG. 3] FIG. 3 shows a photograph illustrating a mechanism portion of a cell processing device used in a production method according to the present invention.
[FIG. 4] FIG. 4 shows a photograph illustrating a state where a closed system portion is attached to a mechanism portion of a cell processing device in the present invention.
[FIG. 5] FIG. 5 shows a block diagram showing another configuration example of a closed system portion of a cell processing device used in a production method according to the present invention.
[FIG. 6] FIG. 6 shows results of expression of monocyte differentiation markers, CD14- and CD45-positive cells, in Example 2.
[FIG. 7] FIG. 7 shows micrographs on days 24 and 28 after establishment of iPS cells and results of expression of GFP carried in a vector and SSEA4 and TRA-1-60 at passages 1 and 3 as confirmed one week after each passage in Example 9.
[FIG. 8] FIG. 8 shows micrographs on days 12 and 16 after establishment of iPS cells and results of expression of GFP carried in a vector and SSEA4 and TRA-1-60 at passages 1 and 3 as confirmed one week after each passage in Example 10.
[FIG. 9] FIG. 9 shows micrographs on days 21 and 28 after establishment of iPS cells and results of expression of GFP carried in a vector and SSEA4 and TRA-1-60 at passages 1 and 3 as confirmed one week after each passage in Example 13.
[FIG. 10] FIG. 10 shows micrographs of differentiation-induced monocytes (on days 17 and 20 of differentiation induction) and results of expression of monocyte differentiation markers, CD14- and CD45-positive cells, in Example 14.
[FIG. 11] FIG. 11 shows micrographs on days 14 and 20 after establishment of iPS cells, and results of expression of GFP carried in a vector and SSEA4 and TRA-1-60 at passage 1 as confirmed one week after the passage in Example 16.
[FIG. 12] FIG. 12 shows a photograph showing an example of a conventional cell processing device.
[FIG. 13] FIG. 13 shows examination results for the methods of coating with a scaffold material in Examples 23 and 24.
[FIG. 14] FIG. 14 shows examination results for the amounts of Sendai virus vector (SeV) in Examples 25 to 27.
[FIG. 15] FIG. 15 summarizes culture medium replacement in the monocyte differentiation processes examined in Examples 28 to 30.
[FIG. 16] FIG. 16 shows examination results for the monocyte differentiation process in Example 28.
[FIG. 17] FIG. 17 shows examination results for the monocyte differentiation process in Example 29.
[FIG. 18] FIG. 18 shows examination results for the monocyte differentiation process in Example 30.

### DESCRIPTION OF EMBODIMENTS

Hereinafter, a method for producing iPS cells according to the present invention will be described in detail.

The production method is a method for producing iPS cells using a cell processing device. The cell processing device includes a closed system portion illustrated in FIG. 1. The closed system portion is configured such that sealed material supply containers (portions indicated by reference numerals 210 to 250 in the example in FIG. 1) and a sealed chamber 100 rotatable for centrifugation are connected via connecting conduits A10. In the production method, the following steps (s1) to (s3) are performed in the stated order in the same single sealed chamber 100 while maintaining closedness of the closed system portion:
Step (s1): A step of separating blood cells from whole blood by centrifugation;
Step (s2): A step of bringing the blood cells into contact with a reprogramming factor; and
Step (s3): A step of establishing iPS cells from the blood cells.

Other processing steps may be added at intervals between the steps (s1), (s2), and (s3), and blood cells may be moved from the sealed chamber to other elements in the closed system portion and then returned to the sealed chamber at intervals between the steps (s1), (s2), and (s3). Blood cells being processed may be aseptically moved to a sealed external container temporarily to perform additional filtering or measurement there, and then returned to the original closed system portion to perform the subsequent step, at intervals between the steps (s1), (s2), and (s3), depending on the need for additional processes such as measurement for quality control. Such additional processes may be manual processes, or may be automatic or semi-automatic processes. In either case, the main steps (s1) to (s3) according to the present invention are automatically performed within the closed system portion according to an instruction from a control unit of the cell processing device (such as Prodigy). The instruction from the control unit is predetermined for the present invention such that the steps (s1) to (s3) are performed automatically and sequentially. By the steps (s1) to (s3), whole blood is processed to provide raw material blood cells, from which iPS cells are established, in one sealed chamber (a sealed chamber for centrifugation) within one closed system.

### (Cell Processing Device)

First, a configuration of each portion of a cell processing device available for the production method will be described.

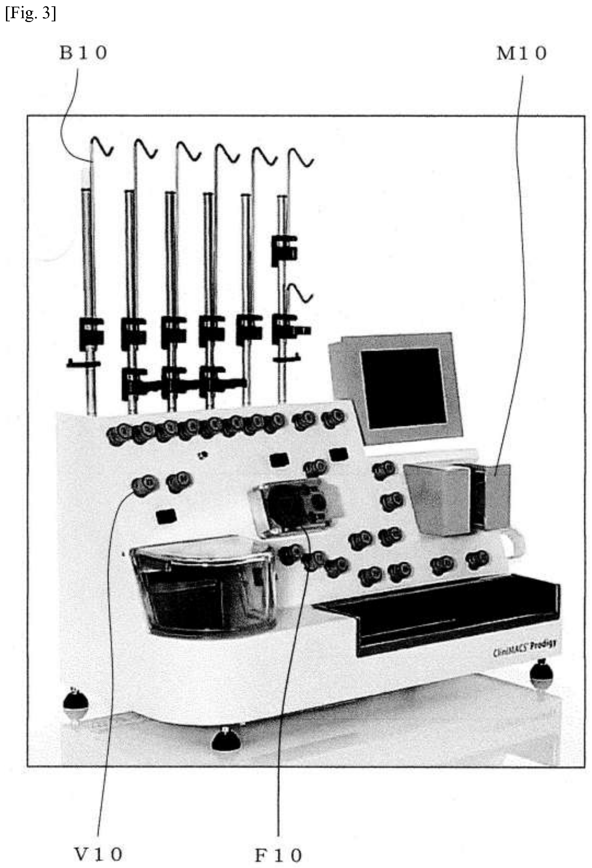

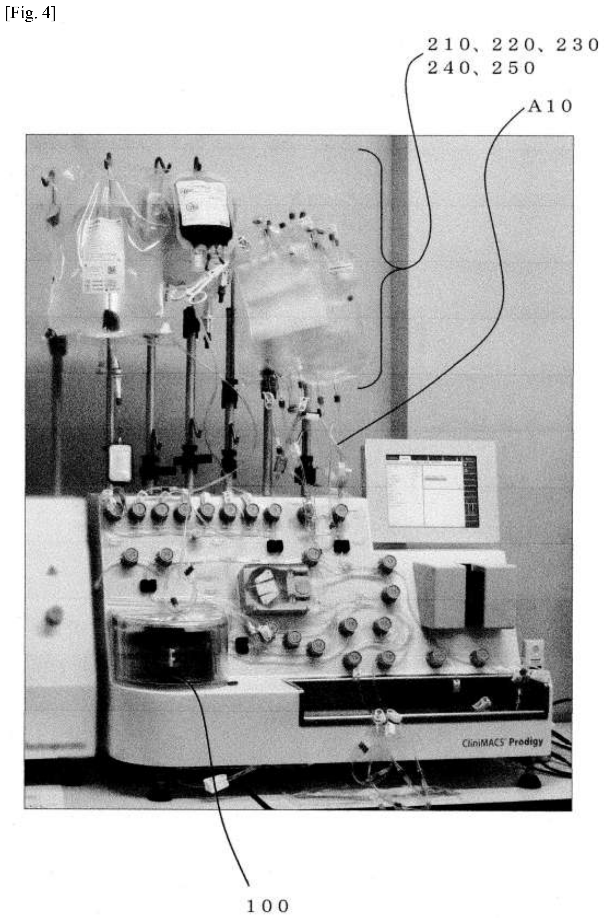

The cell processing device itself may be a conventionally known device as shown in FIG. 12. The cell processing device includes a closed system portion illustrated in FIG. 1 and a mechanism portion illustrated in FIG. 3. In a disposable embodiment, the closed system portion is attached to and detached from the mechanism portion. FIG. 4 illustrates a state where the closed system portion is attached to the mechanism portion. The mechanism portion is automatically operated according to a predetermined control program (computer program) and acts on the closed system portion to perform the steps (s1) to (s3) sequentially and automatically.

A preferred cell processing device is CliniMACS Prodigy (registered trademark) manufactured by Miltenyi Biotec, also shown in FIG. 12. CliniMACS Prodigy is described in detail in, for example, Patent Literature 3. Hereinafter, the CliniMACS Prodigy will also be simply referred to as Prodigy.

Hereinafter, the production method will be described with reference to Prodigy as a cell processing device.

### (Mechanism Portion of Cell Processing Device)

The mechanism portion includes portions other than the closed system portion, such as various actuators, a magnetic separation device, a control unit computer (including a computer program), a power source, a gas supply source, a heat source, a display device, a support portion, and a housing portion. Among the actuators, pinch valves V10 and a peristaltic pump F10 shown in FIGS. 1 and 3 and a motor (not shown) that rotates the sealed chamber 100 are important.

The pinch valves V10 are a kind of electromagnetic valves that block the connecting conduits (soft tubes) of the closed system portion by pressing the connecting conduits from the outside and allow communication of the connecting conduits by stopping pressing.

The peristaltic pump F10, also called tube pump, is a pump that moves a fluid in the connecting conduits (soft tubes) by displacing the portion of the conduits to be pressed from the outside.

The pinch valves V10 and the peristaltic pump F10 can move materials in the conduits from the outside as intended while maintaining closedness of the closed system portion.

The control unit (including a computer and a computer program) of the mechanism portion opens and closes a particular one of the connecting conduits A10 by selectively operating the relevant pinch valve V10, moves essential materials and blood cells being processed in the closed system by operating the peristaltic pump F10, performs centrifugation by rotating the sealed chamber 100, and perform the steps of the production method sequentially. The computer program executed in the computer of the control unit can predetermine the time of opening and closing of any of the pinch valves, the operation period and feeding rate of the peristaltic pump, the operation period and relative centrifugal force of the sealed chamber, and the like to be adapted to the present invention by parameter input and modification. Thus, when the sealed material supply or empty containers necessary for the respective steps are placed as the closed system portion and the predetermined computer program is executed, the steps of the novel production method according to the present invention are performed automatically and sequentially. Execution of the computer program (that is, operation of Prodigy) can be temporarily stopped by predetermination or a stop instruction inputted by a stop switch or the like, and can also be arbitrarily restarted. Thus, manual processes (sample extraction for quality control, filtering) can be additionally performed during the steps or at intervals between the steps.

### (Closed System Portion: Sealed Material Supply Containers)

A number of the sealed material supply containers may be prepared corresponding to the number of types of materials used for processing cells to be produced. The number of types of materials used may differ from the number of sealed containers due to the materials being mixed in advance. A plurality of the sealed containers may be prepared to store the same material required in large amounts. The volume of the sealed container is not particularly limited and varies according to the material, but is about 20 to 3000 ml.

In FIG. 1, as an example for illustration, the sealed container 210 storing whole blood diluted with PBS (for example, a mixture of whole blood and PBS in a volume ratio of 1:1), the sealed container 220 storing a liquid culture medium, the sealed container 230 storing a centrifugation medium, the sealed container 240 storing a fluid that contains a reprogramming factor, and the sealed empty container 250 used as a temporary location for movement of materials are respectively connected to the sealed chamber 100 via the connecting conduits. However, the connection is not limited to the example in FIG. 1, and sealed containers storing materials in accordance with the steps may be connected as a part of the closed system for supplying them. The materials will be described later.

The configuration of sealed material supply containers is not particularly limited and may refer to sealed containers used in conventional cell processing devices such as Prodigy. Preferred examples include soft bags made of flexible films or flexible sheets, and the like. The flexible film is a film that has flexibility to an extent that it can deform according to the amount of contents in the bag. The flexible film may be gas permeable, allowing the permeation of O₂ and CO₂ necessary for cell culture. In Prodigy, a required number of bags as sealed material supply containers are hung from supports B10 shown in FIG. 3 and used as shown in FIG. 4.

### (Closed System Portion: Sealed Chamber with Centrifugation Function)

The sealed chamber 100 is configured to be rotatable for centrifugation while maintaining the closed system, specifically, in a state where the connecting conduits A10 are connected, and even while performing material inflow or outflow. Centrifugation may be based on density gradient centrifugation. A centrifugation medium (described later) necessary for performing density gradient centrifugation may be supplied from the sealed material supply container to the sealed chamber.

Although the material used for fabricating the sealed chamber is not particularly limited, examples thereof include typically used materials, such as glass; polymer compounds such as polystyrene, polycarbonate, polyethylene terephthalate, polyvinylidene fluoride, polyethylene, polypropylene, and polyethylene methacrylate; ceramics; and metals.

### (Centrifugation Mechanism)

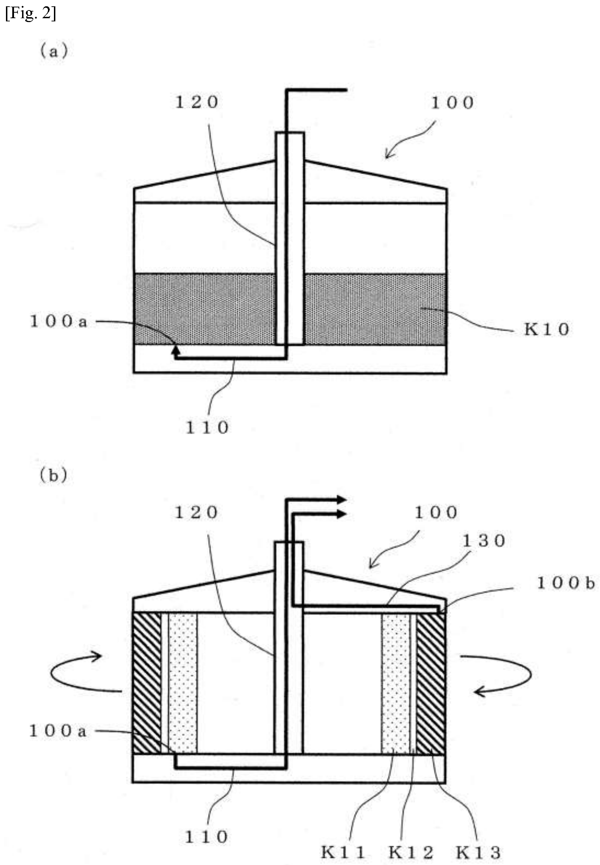

FIG. 2 shows a schematic diagram for describing a manner of centrifugation in a sealed chamber of Prodigy. The shape defined by the side wall surface (inner wall surface) surrounding the internal space of the sealed chamber is approximately a cylindrical shape. The internal space has a support in the center, the inside of which is provided with a flow path. As shown in FIG. 1, a geometrical rotation center line y1 of the cylindrical shape corresponds to a center axis line of rotation movement in centrifugation in the sealed chamber. The bottom surface area of the cylindrical shape is about 90 to 200 cm². The actual inner diameter of the cylindrical shape (the inner diameter of the sealed chamber) may be, but is not limited to, about 12 or 14 cm, for example. In centrifugation, the relative centrifugal force acting on contents in the sealed chamber is 100 to 500 G, and an exemplary relative centrifugal force is 400 G.

As shown in FIGS. 2(a) and 2(b), an opening 100a for material inflow and outflow is provided at a position displaced from the side wall surface to the center at a predetermined distance at the bottom of the sealed chamber 100. The opening 100a is connected to the external connecting conduit shown in FIG. 1 via a flow path 110 passing through the bottom and a central support 120. On the other hand, an opening 100b for material outflow is provided at a position close to the side wall surface at the top of the sealed chamber 100. The opening 100b is in communication with the external connecting conduit shown in FIG. 1 via a flow path 130 at the top.

In centrifugation, first, as shown in FIG. 2(a), an object to be centrifuged (such as whole blood) K10 and, as necessary, a centrifugation medium are injected into the sealed chamber 100 via the flow path 110. Then, as shown in FIG. 2(b), the entire sealed chamber is rotated at a rotational speed necessary for centrifugation. In density gradient centrifugation, this rotation separates an object in the sealed chamber into a plurality of fractions differing in density. In the example in FIG. 2(b), the object is separated into a fraction K11 mainly containing a centrifugation medium, a fraction K12 mainly containing peripheral blood mononuclear cells (PBMC) which are blood cells, and a fraction K13 mainly containing erythrocytes in the order from the inside. With the separated state maintained (specifically, with the sealed chamber rotated), the fraction K11 is discharged from the bottom opening 100a, the fraction K13 is discharged from the top opening 100b, and the fraction K12 mainly containing PBMC remains in the sealed chamber, thereby completing separation by centrifugation (isolation) of the target. In the above manner, the target fraction separated by centrifugation can remain in the sealed chamber, and the other fractions can be discharged from the sealed chamber. Also, only the target fraction separated by centrifugation can be discharged from the sealed chamber as needed. Such a series of processing steps are automatically carried out by operation of drive motors for the pinch valves, peristaltic pump, and sealed chamber according to a predetermined instruction from the control unit.

The volume of the sealed chamber is not particularly limited, but is preferably about 300 to 700 ml, for example. Also, various volumes of the sealed chambers may be prepared according to the scale of processing, such as a regular-sized sealed chamber having a volume of 400 ml and a large-sized sealed chamber having a volume of 600 ml.

The amount of the fluid centrifuged at one time in the sealed chamber is not particularly limited, but is typically about 10 to 250 ml, preferably about 30 to 130 ml.

### (Closed System Portion: Connecting Conduit)

As the tubular member that constitutes the connecting conduit A10, a tube (soft tube) made of a soft material (such as silicone or vinyl chloride) can be preferably used, because the above-mentioned pinch valves and peristaltic pump are used in Prodigy. The connecting conduit may include a pipe member made of a hard material in a portion such as a joint.

### (Closedness or Sealability)

Closedness or sealability in the inside of the closed system portion, sealed containers, and sealed chamber does not only refer to a state where the inside is maintained airtight or liquidtight, but also to a state where the inside is separated from the outside to an extent that microorganisms or viruses cannot enter from the outside, i.e., to an extent that sterility in the inside is maintained. For example, a sealed chamber, in which porous filters (e.g., a pore size of about 0.2 µm or less, particularly about 0.1 to 0.2 µm) are provided that allow permeation of a fluid (particularly gases) but do not allow permeation of bacteria or viruses, allows permeation of outside air through the porous filter and the entry of the outside air into the sealed container, but bacteria and viruses cannot enter the container, and the sterility within the closed system portion is maintained, thus having sealability. A container whose wall is a gas-permeable membrane that prevents permeation of bacteria, viruses, and the like but allows permeation of O₂ gas molecules and CO₂ gas molecules also has sealability. Thus, similarly, a "closed system" does not only refer to a system that is closed airtight or liquidtight, but also to a system whose inside is separated from the outside to an extent that microorganisms or viruses cannot enter from the outside.

Next, iPS cells to be produced, and raw materials and materials necessary for the production will be described.

### (Induced Pluripotent Stem Cells)

In this specification, "induced pluripotent stem cells (iPS cells)" are cells obtained by reprogramming mammalian somatic cells or undifferentiated stem cells by introducing reprogramming factors. Induced pluripotent stem cells can differentiate into tissues and cells with a variety of different morphologies and functions in the body, and have the ability to differentiate into cells of any of the three germ layers (endoderm, mesoderm, and ectoderm).

In this specification, the iPS cells may be cells derived from patients. Generating iPS cells from patient-derived somatic cells for clinical use could be an effective way to minimize the risk of rejection.

The production method can also be used for production of any currently existing iPS cells.

In this specification, the induced pluripotent stem cells may be cells derived from patients with hereditary diseases. Since cells obtained through induction of differentiation of pluripotent stem cells derived from patients with hereditary diseases can serve as disease models that reflect the pathology of such diseases, the cells are suitable for applications such as screening of therapeutic or preventive agents for the diseases. Alternatively, by repairing the gene through genome editing, such as the CRISPR-Cas system, in pluripotent stem cells derived from patients with hereditary diseases, and then differentiating them into target cells, it is also possible to use the cells as therapeutic medicament for the diseases.

### (Whole Blood)

In this specification, "whole blood" refers to blood itself collected from a subject such as a human, from which hemocytes or the like have not been separated. Also, in this specification, "whole blood" may include the whole blood mixed and diluted with a suitable buffer solution or the like; or the whole blood to which an additive such as a blood coagulation inhibitor (such as heparin, EDTA, or citric acid) or a protease inhibitor is added. The buffer for diluting whole blood is not particularly limited as long as it does not cause hemolysis or the like in hemocyte components or the like in the whole blood, and examples thereof include phosphate buffer solutions (PBS) and saline.

### (Amount of Whole Blood Supplied)

In the present invention, the amount of whole blood supplied to the sealed chamber is not particularly limited, but is typically about 5 to 100 ml, preferably about 10 to 50 ml, more preferably about 10 to 40 ml, still more preferably about 10 to 20 ml. Whole blood may be stored in the sealed material supply container as collected and then sent to the sealed chamber, or may be stored in the sealed material supply container as diluted with the above-mentioned buffer solution or the like and then sent to the sealed chamber.

### (Blood Cells)

In this specification, "blood cells" refer to cells in all stages from hematopoietic stem cells through hematopoietic progenitor cells (including pluripotent hematopoietic progenitor cells and unipotent hematopoietic progenitor cells) and finally to functional blood cells. Examples of the blood cells include peripheral blood mononuclear cells (PBMC) and cord blood mononuclear cells (CBMNC).

### (Lymphoid Cells)

In this specification, "lymphoid cells" refer to cells including CD34-positive hematopoietic progenitor cells (lymphoid progenitor cells) and their differentiated cells such as B cells, NK cells, and T cells.

In this specification, the species from which the blood cells are derived is not particularly limited, and the preferred species from which the blood cells are derived is human.

In this specification, unless otherwise specified, the term "cell" includes a "cell population". The cell population may be constituted by one type of cell, or two or more types of cells.

In this specification, "processing" cells means subjecting cells to treatments such as culturing of the cells, dilution of a solution containing the cells, washing of the cells, and separation of the target cells from a solution containing the cells. Also, it also means subjecting cells to chemical treatment, modification of biological properties, combination with non-cellular components, or genetic engineering modification for the purpose of artificial proliferation and differentiation of cells, establishment of cell lines, activation of cells, or the like.

### (Liquid Culture Medium)

The liquid culture medium sent from the sealed containers can be used not only for culturing cells, but also for various purposes in the producing process, such as washing of cells and dilution of chemical solutions. Examples of the liquid culture medium that can be used in the present invention include the following.

A liquid culture medium is not particularly limited, but examples thereof include Essential8 culture medium (CTS^{™} Essential 8^{™} Medium, Essential 8^{™} Medium, Essential 8^{™} Flex Medium, Essential 6^{™} Medium) (Thermo Fisher Scientific Inc.), StemFit (registered trademark) AK02 culture medium (Ajinomoto Co., Inc.), StemFit (registered trademark) AK03 culture medium (Ajinomoto Co., Inc.), StemFit (registered trademark) Basic03 culture medium, CTS (registered trademark) KnockOut SR XenoFree Medium (Gibco), mTeSR1 culture medium, TeSR1 culture medium (Stem Cell Technologies), Iscove's modified Dulbecco's medium (GE HealthCare Technologies Inc.), Improved MEM (Thermo Fisher Scientific Inc.), and the like. These culture media can also be used for culture under feeder-free and xeno-free conditions. Also, examples thereof include, but are not limited to, MSCBM-CD, MSCGM-CD (both are manufactured by Lonza), and mixtures thereof.

Physiologically active substances, nutritional factors, and the like necessary for cell survival or proliferation can be added as needed to the culture medium.

Furthermore, antibiotics such as kanamycin, streptomycin, penicillin, or hygromycin may be added to the culture medium, if necessary.

In this specification, known serum can be used in the culture medium.

In this specification, the culture medium may or may not contain serum substitutes as well as serum.

### (Scaffold Material)

In this specification, the "scaffold material" refers to a material or substrate that functions as a scaffold for cells in cell culture. The scaffold material is not particularly limited as long as it can be used for adherent culture or floating culture using a scaffold material (in other words, it may be free in the culture medium), but examples thereof include those containing or made of synthetic resin, and those made of flexible materials such as collagen. Microcarriers may also be used as scaffold materials. As an example, the scaffold material may include atelocollagen. The scaffold material may be produced using a known method, or a commercially available product may be used. Examples of commercially available products include Cytodex-1 (GE Healthcare) and Corning (registered trademark) Low Concentration Synthemax (registered trademark) II Microcarriers (Corning).

The scaffold material may contain or consist of an extracellular matrix, for example, a basement membrane preparation (e.g., Matrigel (manufactured by Corning) or Geltrex matrix (manufactured by Thermo Fisher Scientific)), fibronectin, laminin or its fragment, entactin, collagen, gelatin, vitronectin, or a combination thereof. The extracellular matrix as exemplified above may be a naturally occurring material, may be a material artificially synthesized by gene recombination technology or the like, or may be a synthetic protein or peptide based on a fragment cleaved by restriction enzyme or the like or based on these bio-derived materials.

Laminin is a heterotrimer molecule composed of α-, β-, and γ-chains, and is an extracellular matrix protein with isoforms that differ in the composition of the subunit chains. Specifically, laminin has about 15 isoforms by heterotrimer combinations from five α-chains, four β-chains, and three γ-chains. Laminin is named by combining the respective numerals for α-chains (α1 to α5), β-chains (β1 to β4), and γ-chains (γ1 to γ3). For example, laminin by combination of α5-, β1-, and γ1-chains is called laminin-511. Laminin may be a naturally occurring material, may be a material artificially synthesized by gene recombination technology or the like, or may be a synthetic protein or peptide based on the laminin.

Laminins or fragments thereof used in the present invention include laminin-111 and a fragment containing its E8 region (e.g., iMatrix-111), laminin-211 and a fragment containing its E8 region (e.g., iMatrix-211), laminin-121 or a fragment containing its E8 region, laminin-221 or a fragment containing its E8 region (e.g., iMatrix-221), laminin-332 or a fragment containing its E8 region (e.g., iMatrix-332), laminin-3A11 or a fragment containing its E8 region, laminin-411 or a fragment containing its E8 region (e.g., iMatrix-411), laminin-421 or a fragment containing its E8 region, laminin-511 or a fragment containing its E8 region (e.g., iMatrix-511 or iMatrix-511 silk), laminin-521 or a fragment containing its E8 region, laminin-213 or a fragment containing its E8 region, laminin-423 or a fragment containing its E8 region, laminin-523 or a fragment containing its E8 region, laminin-212/222 or a fragment containing its E8 region, and laminin-522 or a fragment containing its E8 region.

Vitronectin may be a naturally occurring material, may be a material artificially synthesized by gene recombination technology or the like, or may be a synthetic protein or peptide based on the vitronectin. Examples of commercially available products include highly available products such as Vitronectin, Human Plasma (manufactured by Sigma-Aldrich), Vitronectin (20-398aa), Human, recombinant, Solution (manufactured by FUJIFILM Wako Pure Chemical Corporation), and Vitronectin (VTN-N) Recombinant Human Protein, truncated (manufactured by Gibco).

Fibronectin may be a naturally occurring material, may be a material artificially synthesized by gene recombination technology or the like, or may be a synthetic protein or peptide based on the fibronectin. Examples of commercially available products include highly available products such as Fibronectin Solution, from Human Plasma (manufactured by FUJIFILM Wako Pure Chemical Corporation) and Retronectin (registered trademark) (manufactured by Takara Bio Inc.).

Although the type of collagen is not particularly limited, type I or type IV collagen can be used, for example. Collagen may be a naturally occurring material, may be a material artificially synthesized by gene recombination technology or the like, and may be a synthetic peptide based on the collagen. Examples of commercially available products include highly available products such as Collagen I, Human (manufactured by Corning) and Collagen IV, Human (manufactured by Corning).

Also, the scaffold material may contain or consist of the above-mentioned synthetic resin and extracellular matrix. Examples of polymers (resins) used for scaffold materials include polystyrene, polyolefin, polyethylene terephthalate, polyether, polyvinyl alcohol, polyvinyl acetal (e.g., polyvinyl butyral), polyester, poly(meth)acrylic acid ester (poly(meth)acrylate), epoxy resin, polyamide, polyimide, polyurethane, polycarbonate, cellulose, dextran, and polypeptide (such as gelatin).

Specific examples of the scaffold material containing or consisting of the synthetic resin and extracellular matrix include known materials such as those described in WO2023/127777, Japanese Unexamined Patent Publication No. 2022-077840, Japanese Unexamined Patent Publication No. 2021-023287, Japanese Unexamined Patent Publication No. 2021-003103, Japanese Unexamined Patent Publication No. 2021-003102, Japanese Unexamined Patent Publication No. 2021-003099, Japanese Unexamined Patent Publication No. 2021-003062, Japanese Unexamined Patent Publication No. 2021-003061, Japanese Unexamined Patent Publication No. 2020-174682, Japanese Unexamined Patent Publication No. 2019-118345, Japanese Unexamined Patent Publication No. 2019-115323, Japanese Unexamined Patent Publication No. 2019-115322, WO2019/131982, WO2019/131981, WO2019/131978, Japanese Unexamined Patent Publication No. 2016-106545, Japanese Unexamined Patent Publication No. 2015-221851, WO2015/098919, WO2009/099555, WO2010/138486, WO2010/138687, WO2010/138702, US2011152455A1, WO2011/014594, WO2011/014605, WO2012/150475, and WO2013/116432.

In one embodiment, the scaffold material containing the synthetic resin and peptide includes a polyvinyl acetal resin, preferably a polyvinyl butyral resin, and a cell-adhesive peptide, or a poly(meth)acrylate resin (poly(meth)acrylic acid ester resin) and a cell-adhesive peptide, as described in Japanese Unexamined Patent Publication No. 2021-23287, Japanese Unexamined Patent Publication No. 2022-077840, WO2023/127777, WO2011/014594, or the like. In this specification, the "cell-adhesive peptide" refers to a peptide confirmed to have cell adhesion activity by the phage display method, the sepharose bead method, or the plate coating method. The cell-adhesive peptide is a peptide composed of preferably at least three, more preferably 3 to 20 (e.g., 4, 5, 6, 7, 8, 9, 10, 11, 12, 13, 14, 15, 16, 17, 18, or 19), still more preferably 3 to 15, particularly preferably 3 to 10, amino acids. The cell-adhesive peptide preferably contains an RGD sequence (Arg-Gly-Asp). Commercially available products of such a scaffold material include Corning (registered trademark) Synthemax (registered trademark) vitronectin substrate (manufactured by Corning).

The method for supplying the scaffold material to the sealed chamber or the like is not particularly limited as long as the desired cells can be suitably processed, and the supply conditions (e.g., the supply timing, the volume, or the like), the type, and the like of the scaffold material can be determined by those skilled in the art as appropriate.

In one embodiment, the above-mentioned scaffold material may be used for coating the inside of a sealed chamber used in the production method according to the present invention before performing at least one of the steps (s1), (s2), (s3), (s4), and (s5). In another embodiment, the above-mentioned scaffold material may be used for coating the inside of the sealed chamber by adding it into a culture medium used in at least one of the steps (s1), (s2), (s3), (s4), and (s5), when the scaffold material used is iMatrix. In another embodiment, the scaffold material may be supplied into the sealed chamber by adding it into a culture medium used in at least one of the steps (s1), (s2), (s3), (s4), and (s5).

### (Centrifugation Medium)

In the step (s1), density gradient centrifugation is useful for separating blood cells from whole blood. In this case, a centrifugation medium for the density gradient centrifugation is supplied from the sealed material supply container to the sealed chamber. Examples of the centrifugation medium include hydrophilic polysaccharides (such as Ficoll^{™}).

In the case of density gradient centrifugation in the step (s1), whole blood (which may be diluted) and the centrifugation medium are supplied to the sealed chamber and subjected to density gradient centrifugation, and thus a fraction containing blood cells remains in the sealed chamber.

### (Reprogramming Factor)

In the step (s2), the pinch valves and peristaltic pump are operated, and a fluid containing a reprogramming factor is sent from the sealed material supply container into the sealed chamber. By the step (s1), the separated blood cells are present in the sealed chamber, and the reprogramming factor is brought into contact with the blood cells. When the reprogramming factor is brought into contact with the blood cells, the sealed chamber may be in a resting state, in a rotated state, or in a combination of these states.

In this specification, examples of "reprogramming factors" include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, ESrrb, Nr5a2, Tbx3, and Glis1, and these reprogramming factors may be used alone or in combination. Any known combination of reprogramming factors can be used.

The reprogramming factor introduced into the somatic cells may be in the form of a protein, a nucleic acid (RNA or DNA) encoding the protein, or an expression vector containing the nucleic acid. When the reprogramming factor is introduced in the form of RNA, the immunogenic RNA introduced into the cell may activate the cell's defense mechanism, and thus RNA may be introduced into the somatic cell to circumvent this defense mechanism.

Examples of expression vectors include viral vectors such as retrovirus, lentivirus, adenovirus, adeno-associated virus, herpes virus, and Sendai virus, as well as plasmid vectors, episomal vectors, artificial chromosome vectors, and transposon vectors (piggyBac, piggyBac, TolII).

Nucleic acids, expression vectors containing the nucleic acids, or proteins (e.g., reprogramming factors) can be introduced into cells using various known methods. Such methods include, for example, calcium phosphate-mediated transfection, electroporation, liposome transfection, lipofection, gene gun, microinjection, viral vector method, virus-like particle method, Agrobacterium method, agroinfiltration method, PEG-calcium method, sonoporation method, lipid nanoparticle method, and the like.

### (Material Constituting Sealed Chamber and Cell Culture)

The material of the sealed chamber of Prodigy contains a portion made of a polycarbonate resin. The main portions (inner side wall surface and inner bottom surface) of the internal space of an actual sealed chamber are surfaces made of a polycarbonate resin. Therefore, these surfaces are non-cell-adhesive and are not suitable for culture of iPS cells which are adherent cells (adherent culture utilizing an inner wall surface). Thus, in a preferred embodiment of the production method, a suitable scaffold material is supplied to the sealed chamber, and the steps (s2) and (s3) are performed in the presence of the scaffold material. The scaffold material may also be supplied to the sealed chamber in the step (s2) for convenience of coating.

In a preferred embodiment of the production method, the scaffold material is adhered to the inner wall surface of the sealed chamber. This allows adherent culture even if the inner wall surface of the sealed chamber is made of a polycarbonate resin, and is therefore preferable. Examples of such a scaffold material include those containing the above-mentioned laminin fragment (e.g., laminin-511 or laminin-332), those containing the above-mentioned cell-adhesive peptide and polyvinyl acetal resin (e.g., a polyvinyl butyral resin), or those containing the above-mentioned cell-adhesive peptide and poly(meth)acrylate resin. In a preferred embodiment, the cell-adhesive peptide contains an RGD sequence (Arg-Gly-Asp).

In a method for supplying the scaffold material, one vial of Synthemax powder (10 ml) can be dissolved by adding 10 ml of sterile water thereto and aseptically sealed in a sealed container, in a safety cabinet, then the sealed container can be removed from the safety cabinet and aseptically connected to Prodigy, and then the scaffold material can be sent into the sealed chamber by the peristaltic pump, kept at 22°C overnight, and used for precoating. When the scaffold material is used in a small amount, it may be mixed with PBS or sterile water for ease of supply. Liquid iMatrix undiluted can be supplied.

### (Other Materials to be Supplied)

In addition to the above-mentioned materials, materials necessary for processing cells may be added as appropriate. Like the above-mentioned materials, the additional materials can also be stored in the sealed containers or the like and connected to the sealed chamber. Other materials to be added include, for example, liquids, powders, additives, release agents, cryoprotectants, and CO₂.

### (Step (s3) of Establishing iPS Cells)

The step (s3) in the production method according to the present invention is a step of establishing iPS cells by culturing blood cells in a liquid culture medium, and the establishment of induced pluripotent stem cells can be confirmed as appropriate by expression or the like of a reprogramming factor (such as Oct3/4, SOX2, Nanog, TRA-1-60, TRA-1-81, SSEA3, SSEA4, or alkaline phosphatase) introduced by a method known per se. The period of performing the step (s3) is not particularly limited as long as iPS cells can be established, but is typically 10 days or more, preferably 14 days or more, for example. The upper limit is also not particularly limited, but is typically 30 days or less, preferably 40 days or less.

Preferred examples of operation of Prodigy to perform the steps (s1) to (s3) are the following steps (e1) to (e15).

(e1) In FIG. 1, diluted whole blood stored in the sealed material supply container 210 (for example, 20 ml of whole blood diluted with 30 ml of phosphate buffered saline (PBS)) is sent to the sealed chamber 100.

(e2) Ficoll, the density gradient centrifugation medium stored in the sealed material supply container 230, is sent to the sealed chamber 100.

(e3) The sealed chamber 100 is rotated to perform density gradient centrifugation, and a fraction mainly containing PBMC remains in the sealed chamber.

(e4) The fraction mainly containing PBMC is sent to the sealed empty container 250. The connecting conduit connected to the sealed container 250 is disconnected by heat sealing, such that the sealed container 250 is aseptically disconnected from Prodigy.

(e5) The sealed container 250 and another sealed container incorporating a filter are aseptically joined, and PBMC stored in the sealed container 250 is put into the upper inlet of the other sealed container incorporating the filter, such that debris (something like a mass of unnecessary substances from cells) is removed through the filter in the sealed chamber.

(e6) A part of the fraction mainly containing PBMC is manually sampled and stained with trypan blue, and the cell count is measured with a cell counter. The steps (e4) to (e6) are not steps essential for the present invention, but are steps preferably performed.

(e7) Optionally, at this time, a scaffold material (such as Synthemax) is sent into the sealed chamber 100 from the sealed material supply container, and the inner wall surface of the sealed chamber is coated with the scaffold material.

(e8) The sealed container incorporating the filter is aseptically connected to the closed system portion manually, the fraction mainly containing PBMC is returned to the sealed chamber 100 by the peristaltic pump, and a fluid containing Sendai virus vector as a reprogramming factor is sent from the sealed material supply container 240 to the sealed chamber 100.

(e9) Sendai virus vector is brought into contact with PBMC in the sealed chamber (two hours).

(e10) A liquid culture medium is added from the sealed material supply container 220 into the sealed chamber.

(e11) Some contents in the sealed chamber are manually sampled, and the cell count is measured. The step (e11) is not a step essential for the present invention, but is a step preferably performed.

(e12) The liquid culture medium in the sealed chamber is reduced, cytokines are added, and cell culture is started (establishment of iPS cells is started).

(e13) Optionally, at this time, the scaffold material is supplied from the sealed material supply container to the sealed chamber 100 and mixed by rotation of the sealed chamber (the mixing conditions are 37°C and about 30 to 60 minutes when the inner wall surface of the sealed chamber is coated).

(e14) Replacement or addition of the liquid culture medium is performed in the sealed chamber.

(e15) 14 to 21 days after the start of cell culture (the start of establishment of iPS cells), colonies (in the case of adherent culture) or spheroids (in the case of floating culture) are found.

### (Expansion Culture)

The production method may further include, after the above-mentioned step (s3) of establishing iPS cells, a step (s4) of carrying out expansion culture of the iPS cells in the sealed chamber from the viewpoint of yield of iPS cells. The step (s4) is performed while maintaining closedness of the closed system portion as in the steps (s1) to (s3). Expansion culture of the iPS cells in the sealed chamber can be carried out by supplying a culture medium suitable for expansion culture to the sealed chamber. The culture medium or the like used in the step (s4) can be selected from the above-mentioned liquid culture media by those skilled in the art as appropriate. In one embodiment, when the established induced pluripotent stem cells are intended to be subjected to expansion culture, an undifferentiated state maintenance factor can be added. In this specification, the "undifferentiated state maintenance factor" refers to a substance that has the effect of suppressing differentiation of induced pluripotent stem cells, and is not particularly limited as long as it is such a substance. Examples of the undifferentiated state maintenance factors that are commonly used by those skilled in the art include bFGF, FGF2, FGF4, FGF8, EGF, Nodal, Activin A, Activin B, TGFβ1, and TGFβ2. The undifferentiated state maintenance factor used in the present invention is preferably isolated. "Isolation" means that an operation to remove factors other than the target component or cells is performed such that the product is no longer in a naturally occurring state.

Although there are no particular limitations to how many times expansion culture is carried out in the step (s4) as long as a desired number of iPS cells can be obtained, expansion culture is carried out preferably about 1 to 5 times, more preferably about 2 to 5 times, in a typical processing operation. The period of performing the step (s4) is not particularly limited as long as a desired number of iPS cells can be obtained, but is typically 1 to 40 days, 3 to 20 days, or 5 to 10 days, for example. When Sendai virus vector used for reprogramming contains a GFP marker, it is preferable to carry out expansion culture until the marker is eliminated.

### (Magnetic Separation Step)

In the production method according to the present invention, when the blood cells to be separated in the above-mentioned step (s1) are the above-mentioned lymphoid cells (e.g., CD34-positive cells (more specifically, hematopoietic progenitor cells), the lymphoid cells required can be isolated from PBMC by separating PBMC from whole blood by centrifugation and then further performing an additional magnetic separation step. In this case, as shown in FIG. 5, a magnetic separation column 300 connected to the sealed chamber 100 via the connecting conduit A10 is used as a part of a configuration of the closed system portion. The magnetic separation column 300 is attached to a magnetic separation unit M10 in the mechanism portion shown in FIG. 3. In FIG. 5, the lymphoid cells to be separated are labeled with magnetic beads in the sealed chamber 100. In the example in FIG. 5, the magnetic beads are sent from the sealed material supply container 260 to the sealed chamber. When a suspension containing the lymphoid cells (with the magnetic beads) is sent from the sealed chamber 100 into the magnetic separation column 300, only the lymphoid cells (with the magnetic beads) are trapped in the magnetic separation column 300 (that is, magnetically separated) by a magnetic field acting from the magnetic separation unit M10. Other blood cells and foreign matters pass through the magnetic separation column 300 and are sent to a bag (not shown) for negative selection. Then, application of the magnetic field by the magnetic separation unit M10 is stopped, the pinch valves are switched, and the lymphoid cells (with the magnetic beads) trapped in the magnetic separation column 300 leave the magnetic separation column 300 and are sent to a bag 310 for positive selection. Then, the pinch valves are switched, and the lymphoid cells (with the magnetic beads) in the bag 310 for positive selection are returned to the sealed chamber 100. The magnetic beads may be actively removed by elimination treatment and subjected to the subsequent step; however, when they are superfine magnetic beads of about 50 nm, for example, such superfine magnetic beads are spontaneously eliminated from the lymphoid cells by continuously processing them in the subsequent step (s2).

### (Magnetic Beads, Elimination of Magnetic Beads)

The process and necessary materials for the magnetic separation method itself can be determined or selected as appropriate with reference to a known technology. Magnetic beads include CliniMACS CD1c (BDCA-1) GMP Biotin, CliniMACS CD3 GMP MicroBeads, CliniMACS CD4 GMP MicroBeads, CliniMACS CD8 GMP MicroBeads, CliniMACS CD14 GMP MicroBeads, CliniMACS CD19 GMP MicroBeads, CliniMACS CD25 GMP MicroBeads, CliniMACS CD34 GMP MicroBeads, CliniMACS 45RA GMP MicroBeads, CliniMACS Prodigy CD45RA XS GMP Kit, CliniMACS CD56 GMP MicroBeads, CliniMACS CD62L GMP MicroBeads, CliniMACS CD133 GMP MicroBeads, CliniMACS CD133 GMP MicroBeads, CliniMACS CD137 GMP Biotin, CliniMACS CD304 (BDCA-4) GMP MicroBeads, CliniMACS Anti-Biotin GMP MicroBeads, CliniMACS TCRα/β GMP Biotin, and CliniMACS TCRα/β GMP Kit.

The method of eliminating magnetic beads may be spontaneous elimination of magnetic beads in the course of cell culture.

Materials for modification with and elimination of magnetic beads may be sent into the sealed chamber as appropriate from the sealed material supply container added as necessary.

In the steps (s1) to (s2), preferred examples of operation of Prodigy that includes magnetic separation of lymphoid cells are as follows, and are performed according to the "T cell transduction process", the magnetic separation program of Prodigy.

(f1) In FIG. 1, 50 ml of diluted whole blood stored in the sealed material supply container 210 (20 ml of whole blood diluted with 30 ml of phosphate buffered saline (PBS)) is sent to the sealed chamber 100.

(f2) 150 ml of Ficoll^{™}, the density gradient centrifugation medium stored in the sealed material supply container 230, is sent to the sealed chamber 100.

(f3) The sealed chamber 100 is rotated to perform density gradient centrifugation, and a fraction mainly containing PBMC remains in the sealed chamber.

(f4) The fraction mainly containing PBMC is sent to the sealed empty container 250.

(f5) The fraction is sent to a labeling column (not shown) in Prodigy, and lymphoid cells in PBMC are labeled with magnetic beads.

(f6) The lymphoid cells (with the magnetic beads) are returned to the sealed chamber and incubated.

(f7) Washing to remove excessive magnetic beads is performed in the sealed chamber.

(f8) The lymphoid cells (with the magnetic beads) are sent to and magnetically trapped in the magnetic separation column.

(f9) The cells are washed in the magnetic separation column.

(f10) Magnetic field application is stopped in the magnetic separation unit.

(f11) The lymphoid cells (with the magnetic beads) are returned to the sealed chamber 100. The magnetic beads are directly subjected to the subsequent reprogramming step due to their extreme fineness.

(f12) A part of the fraction is manually sampled and stained with trypan blue (Gibco, Trypan Blue Strain (0.4%), 15250-061, filtered), and the cell count is measured with a cell counter. The step (f12) is not a step essential for the present invention, but is a step preferably performed.

(f13) Optionally, at this time, a scaffold material (such as Synthemax) is sent into the sealed chamber 100 from the sealed material supply container, and the inner wall surface of the sealed chamber is coated with the scaffold material.

(f14) A fluid containing Sendai virus vector as a reprogramming factor is sent from the sealed material supply container to the sealed chamber 100.

(f15) Sendai virus vector is brought into contact with the lymphoid cells (with the magnetic beads) in the sealed chamber (two hours).

(f16) A liquid culture medium is added from the sealed material supply container into the sealed chamber.

(f17) Some contents in the sealed chamber are sampled, and the cell count is measured.

(f18) The liquid culture medium in the sealed chamber is reduced, cytokines are added, and cell culture is started (establishment of iPS cells is started).

(f19) Optionally, at this time, the scaffold material is supplied from the sealed material supply container into the sealed chamber and mixed (the mixing conditions are 37°C and about 30 to 60 minutes, for example, when the inner wall surface of the sealed chamber is coated).

(f20) Replacement or addition of the liquid culture medium is performed in the sealed chamber.

(f21) 14 to 21 days after the start of cell culture (the start of establishment of iPS cells), colonies (in the case of adherent culture) or spheroids (in the case of floating culture) are found.

### (Quality Evaluation of iPS Cells)

After the step (s3) or (s4) of the production method according to the present invention, the quality of the obtained iPS cells may be evaluated using a known method.

### (Method for Producing Differentiated Cells)

Next, a method for producing differentiated cells according to the present invention will be described.

The method for producing differentiated cells includes the steps (s1) to (s3) in the above-described method for producing iPS cells according to the present invention, and further includes, after these steps of the production method, a step (s5) of inducing differentiation of the iPS cells. The step (s5) is performed while maintaining closedness of the closed system portion, as in the steps (s1) to (s3) and the step (s4). In the step (s5), materials necessary for differentiation induction are supplied from sealed material supply containers (not shown) into the sealed chamber, and the step (s5) is performed in the sealed chamber. The sealed containers for supplying the materials necessary for differentiation induction are not shown in FIGS. 1 and 5.

The step (s5) may be performed without performing the step (s4) of carrying out expansion culture of the iPS cells after the step (s3) in the above-mentioned method for producing iPS cells according to the present invention, or may be performed after performing the step (s4) of carrying out expansion culture of the iPS cells after the step (s3).

### (Differentiated Cells)

In this specification, "differentiated cells" refers to cells or organoids obtained by inducing differentiation of induced pluripotent stem cells. Cells obtained may be undifferentiated cells such as stem cells and progenitor cells, or terminally differentiated cells. In this specification, a term "differentiated cell" may be used as a term that encompasses both an undifferentiated cell and a terminally differentiated cell obtained by inducing differentiation of induced pluripotent stem cells. In this specification, the term "undifferentiated cells" means cells that do not reach terminal differentiation in the cell lineage, and examples of the undifferentiated cells include stem cells other than pluripotent stem cells, and progenitor cells. Examples of stem cells or progenitor cells include ectodermal cells such as neural crest cells, neural stem cells, neural progenitor cells, glial progenitor cells, retinal stem cells, corneal stem cells, keratinocyte epidermal stem cells, melanocyte stem cells, and mammary stem cells, mesodermal cells such as hematopoietic progenitor cells, myeloid stem cells, lymphoid stem cells, B progenitor cells, T progenitor cells, mesenchymal stem cells, cardiac stem cells, cardiac progenitor cells, vascular endothelial progenitor cells, vascular pericytes, platelet progenitor cells, skeletal muscle stem cells, adipose stem cells, and kidney progenitor cells, and endodermal cells such as hepatic stem cells, liver progenitor cells, intestinal stem cells, and airway stem cells.

In this specification, "terminally differentiated cells" refers to cells that have reached terminal differentiation in a cell lineage. Examples of terminally differentiated cells include, but are not limited to, osteoblasts, chondrocytes, adipocytes, hepatocytes, hepatic mesothelial cells, bile duct epithelial cells, hepatic stellate cells, hepatic sinusoidal endothelial cells, Kupffer cells, pit cells, vascular endothelial cells, blood cells, pancreatic duct epithelial cells, pancreatic duct cells, centroacinar cells, acinar cells, islets of Langerhans, cardiac muscle cells, fibroblasts, smooth muscle cells, type I alveolar epithelial cells, type II alveolar epithelial cells, Clara cells, ciliated epithelial cells, basal cells, goblet cells, neuroendocrine cells, Kulchitsky cells, renal tubular epithelial cells, urothelial cells, columnar epithelial cells, glomerular visceral epithelial cells, glomerular endothelial cells, podocytes, mesangial cells, nerve cells, and glial cells. Examples of white blood cells include lymphocytes, granulocytes, and monocytes.

In an embodiment, the cells or organoids (target cells or organoids) obtained by inducing differentiation of induced pluripotent stem cells are neural crest cells, neural progenitor cells, nerve cells, cerebral cortical organoids, hematopoietic progenitor cells, platelets, T cells, or cardiac muscle cells.

### (iPS Cell Differentiation-Inducing Substance)

In this specification, the "differentiation-inducing substance" refers to a substance that can induce differentiation from induced pluripotent stem cells into the differentiated cells or organoids described above. The differentiation-inducing substance may be a substance known per se, or may be appropriately selected from substances that are commonly used for inducing differentiation of desired differentiated cells or organoids. Specifically, examples thereof include the substances described below.

The fluid used as a medium for containing the reprogramming factors, undifferentiated maintenance factors, and differentiation-inducing substances is not particularly limited, but preferred examples thereof include buffer solutions, culture media, and cryoprotectants such as dimethyl sulfoxide (DMSO) and glycerin.

### (Differentiation Induction Method)

In the production method according to the present invention, a known method can be used as a differentiation induction method to obtain target cells or organoids. For example, differentiation of pluripotent stem cells into neural crest cells can be induced using methods such as those described in Fukuta M. et al., PLoS One, 2014, 9(12): e112291 and Kamiya D, et al., NPJ Regen Med., 2022 Sep 15;7(1):47, and the like. Specifically, pluripotent stem cells can be differentiated into neural crest cells by seeding them into a culture vessel and culturing them in adherent culture (floating culture using a scaffold material), and then culturing them in adherent culture (floating culture using a scaffold material) in a culture medium containing a TGFβ inhibitor and a GSK3β inhibitor.

It is also possible to produce, from neural crest cells, cells such as mesenchymal stem cells, neural progenitor cells, nerve cells, glial cells, bone cells, chondrocytes, corneal cells, and melanocytes. For example, differentiation into these cells can be induced based on methods such as those described in Fukuta M. et al., PLoS One, 2014, 9(12): e112291, Horikiri T. et al., PLoS One, 2017, 12(1): e0170342, and Kamiya D, et al., NPJ Regen Med., 2022 Sep 15;7(1):47, and the like. Specifically, for example, neural progenitor cells and nerve cells can be obtained by seeding neural crest cells onto a fibronectin-coated plate, replacing the culture medium with DMEM/F12 supplemented with N-2 Supplement, BDNF, GDNF, NT-3, and NGF, and culturing the cells at 37°C under 5% CO₂ for about 14 days. Alternatively, neural progenitor cells and nerve cells can be obtained by seeding neural crest cells onto a plate and culturing the cells in the CDM containing SB431542 and CHIR99021 for one day, then replacing the medium with Neurobasal medium containing B-27 Supplement, N-2 Supplement, L-glutamine, Penicillin/Streptomycin, BDNF, GDNF, NT-3, and NGF, and culturing the cells at 37°C under 5% CO₂ for about 35 days.

The differentiation of cells into mesenchymal stromal cells can be induced, for example, using the following method. Neural crest cells are seeded in a culture vessel and cultured for 1 day in the CDM medium containing SB431542 and CHIR99021. After one day, the medium is replaced with αMEM containing FBS. About 14 days after the start of differentiation induction, mesenchymal stromal cells can be obtained.

Examples of a method for differentiating pluripotent stem cells into T cells include a method including (1) a step of differentiating pluripotent stem cells into hematopoietic progenitor cells and (2) a step of differentiating the hematopoietic progenitor cells into T cells. The step (1) may be a step of culturing pluripotent stem cells in a culture medium for inducing hematopoietic progenitor cells, as described, for example, in WO2013/075222, WO2016/076415, Liu S. et al., Cytotherapy, 17 (2015); 344-358, and the like. The step (2) may be (2-1) a step of inducing CD4CD8 double-positive T cells from hematopoietic progenitor cells, or (2-2) a step of inducing CD8 positive T cells from CD4CD8 double-positive T cells, as described in WO2016/076415 and the like.

Examples of a method for inducing differentiation of pluripotent stem cells into cardiomyocytes include the methods described in WO2015/141827 and Laflamme MA and Murry CE, Nature. 473(7347):326-35 (2011). Other methods include, for example, a method of producing cardiomyocytes by forming embryoid bodies from induced pluripotent stem cells through floating culture, a method of producing cardiomyocytes in the presence of a substance that suppresses BMP signaling (WO2005/033298), a method of producing cardiomyocytes by sequentially adding Activin A and BMP (WO2007/002136), and a method of producing cardiomyocytes in the presence of a substance that promotes activation of the canonical (classical) Wnt signaling pathway (WO2007/126077). Typically, examples of marker proteins for cardiomyocytes include NKX2.5 (a cardiac muscle-specific transcription factor) and TNNT2 (troponin T), while examples of marker proteins for cardiac progenitor cells include KDR (a receptor for vascular endothelial growth factor (VEGF)) and ISL1 (a LIM homeodomain transcription factor).

Differentiation into monocytes can be induced by a method described in Di Cui., et al. Frontiers in Cell and Developmental Biology; vol 9, Article 656867 April 2021.

Organoids can also be produced using multiple types of cells. For example, in the case of hepatic organoids, as described in WO2013/047639 or the like, hepatic organoids can be produced by inducing hepatic progenitor cells (organ cells), mesenchymal stem cells, and vascular endothelial cells from pluripotent stem cells, and subjecting the mixture thereof to floating culture.

In the production method of the present invention, the culture may be conducted under feeder-free conditions and/or xeno-free conditions over the entire period of the culture or during a part of the period of the culture. From the viewpoint of a clinical application, it is preferable to conduct the differentiation induction method of the present invention under feeder-free conditions and xeno-free conditions throughout the entire period.

The production method of the present invention may include a step of collecting obtained cells or organoids of interest. The collected cells may be cryopreserved using a cell cryopreservation solution. The number of cells collected in a container may be counted using a cell counter, and the cells may be labeled with an antibody against cell surface marker and then purified using flow cytometry, mass cytometry, a magnetic cell sorting method, or the like.

In the cell production method according to the present invention, undifferentiated cells may be removed as appropriate. The method for removing undifferentiated cells is not particularly limited as long as it can remove cells other than cells produced using the cell production method according to the present invention, and the undifferentiated cells can be removed by adding a known undifferentiated cell removing agent or the like to the culture medium (e.g., Di Mao., et al. Angewandte Chemie International Edition; 9 January 2017; Ben-David, U., et al. Cell Stem Cell, 12, 167 (2013); WO2019/187918; Japanese Unexamined Patent Publication No. 2016-93178; Yoshiki Nakashima, et al., Molecular Therapy Vol. 26 No. 7 July 2018, and the like).

Quality testing may also be carried out as appropriate to determine whether the cells, organoids, or the like obtained by the production method of the present invention are desired. The test items in the quality testing are not particularly limited, but include basic tests such as the morphology of cells and organoids, the presence or absence of cell surface marker expression, sterility tests, endotoxin tests, and evaluation of cell viability, and various testing devices are available for each item.

### EXAMPLES

In the examples shown below, the steps (s1) to (s5) of the production method according to the present invention were performed using the following reagents, devices, and materials, and the obtained iPS cells and differentiated cells (monocytes) were evaluated.

The closed system portion of Prodigy used has the same configuration as in FIG. 1, and the main portion of the sealed chamber is made of a polycarbonate resin. The closed system portion was attached to a mechanism portion shown in FIG. 3 to be operable as shown in FIG. 4.

Operation of Prodigy to perform the steps (s1) to (s3) is the same as in the steps (e1) to (e15) or the steps (f1) to (f25) illustrated above as preferred examples of operation of Prodigy.

Although conditions such as supplied materials and their amounts differ between the examples, basic operation of Prodigy is the same as in the steps (e1) to (e15) or the steps (f1) to (f21) above.

In the step (s4) of carrying out expansion culture of the established iPS cells and the differentiation induction step (s5), the basic operation method of Prodigy is also the same as in the steps (s1) to (s3), although conditions such as materials to be supplied into the sealed chamber and their amounts differ.

### Reagents

Whole blood: Peripheral blood from a healthy volunteer donor: Whole blood, received on the previous day of the experiment and stored at room temperature under shading horizontally.
Leukocyte removal filter: Sepacell, RZ-2000N, Asahi Kasei
Ficoll-Paque PLUS (GE Healthcare, Chicago, IL, USA)
2% HSA: CSL Behring K.K., Albuminar 25% I.V. Injection 12.5 g/50 mL
HSC Brew GMP Medium: Miltenyi Biotec, 170-076-310
Six cytokines used for reprogramming
   SCF: Recombinant Human SCF (FUJIFILM Wako Pure Chemical, 197-15511)
   TPO: Recombinant Human TPO (FUJIFILM Wako Pure Chemical, 207-17581)
   Flt-3L: Recombinant Human Flt3L (FUJIFILM Wako Pure Chemical, 061-05391)
   IL-6: Recombinant Human IL-6 (FUJIFILM Wako Pure Chemical, 098-06041)
   IL-3: Recombinant Human IL-3 (FUJIFILM Wako Pure Chemical, 090-05761)
   G-CSF: Recombinant Human G-CSF (FUJIFILM Wako Pure Chemical, 072-06101)
iMatrix-511: MATRIXOME
iMatrix-322: MATRIXOME
An acrylic resin containing a peptide having an RGD sequence
Synthemax II-SC Corning, 3535, 10 mg
Laminin 521: BLA-LN521-05 Human recombinant laminin 521, 500 µg
SeV: SRVTM iPSC-4, TOKIWA-Bio Inc., carrying GFP.
StemFit (registered trademark) AK03N: Ajinomoto Healthy Supply Co., Inc.
TrypLE: TrypLETM Select (1X) Gibco, 12563-029
0.5 mmol/L-EDTA/PBS solution: Nacalai Tesque, Inc.
ROCK inhibitor: CultureSure 10 mmol/L Y-27632 solution, Animal-derived-free, Wako Pure Chemical, 035-24593
StemSpan-AOF: Stemcell Technologies, British Columbia, Canada
BMP4: 314-BP-010
VEGF: R&D Systems, Minneapolis, MN, 293-VE-050
CHIR: Miltenyi Biotec, 130-103-926
Essential 8 Medium: Gibco, A1517001

### Materials

Blood bag (FLEXBOY (registered trademark) BAG 50 ml (LUER LOCKS, Sartorius Stedim Biotech))
Reagent bag: Miltenyi Biotec, 20 mL Reagent Bag, 170-076-631
Terumo separation bag
Microbeads: CliniMACS CD34 GMP MicroBeads, Miltenyi Biotec

### Devices

Cell processing device: CliniMACS Prodigy, Miltenyi Biotec
Sterile connecting device: TERUMO TSCD-II
5% CO₂ incubator: Panasonic Healthcare, MCO-170AICUVH-PJ
Microscope: Inverted routine microscope, trinocular lens barrel phase contrast set (precentered) CKX41, manufactured by Olympus Corporation
Automatic cell counter: CountessII, Thermo Fisher Scientific
Flow cytometry: SA3800 Spectral Cell Analyzer, Sony

### Example 1

### (1) Attachment of Tubing Set

On the previous day of SeV infection, the bar code of a tubing set (type: TS730) of Prodigy was read, and the ACC program was selected and operated according to the operation screen. In a safety cabinet, tube loosening and breakage during transportation of the tubing set including a sealed chamber of Prodigy were manually confirmed, and the tubing set was tightened and assembled to Prodigy.

### (2) Preparation of Starting Material (Separation of PBMC from Whole Blood)

A blood bag was aseptically filled with 70 ml of whole blood manually in the safety cabinet. A bag was aseptically filled with 150 ml of Ficoll-Paque, and a bag filled with 2% HSA was aseptically filled with HSC Brew. These bags were aseptically connected to Prodigy via tubes using a sterile connecting device and hung from Prodigy. On the screen of Prodigy, the density gradient program (LP-34) of the Adherent Cell Culture program was selected, and the conditions were determined and inputted, and the program was started. With the program, whole blood was moved from the blood bag to the chamber, and Ficoll was then put into the chamber from the hung Ficoll bag and centrifuged to obtain PBMC. With the program of Prodigy, PBMC was moved from the chamber to a bag. The program was temporarily stopped, PBMC contained in the bag was manually put into the upper opening of another bag incorporating a filter by aseptic connection using a sterile connecting device (TERUMO TSCD-II), and the debris was removed from PBMC through the filter in the bag.

### (3) Incubation of PBMC

With the program, the inside of the chamber was washed with 500 ml of HSC Brew. The bag containing PBMC suspended in 2% HSA HSC Brew was detached, and in the safety cabinet, PBMC was moved to a cell expansion bag containing six cytokines (the protocol described in the website of the CiRA Foundation: SFC (final concentration 50 ng/mL), TPO (final concentration 10 ng/mL), Fit3L (final concentration 20 ng/mL), IL-6 (final concentration 50 ng/mL), IL-3 (final concentration 20 ng/mL), G-CSF (final concentration 10 ng/mL), 108 µl, 20 mL/min). PBMC was cultured in a CO₂ incubator. The Prodigy chamber was washed with the program. In the safety cabinet, a bag was manually filled with 0.35 ml × 3 tubes (3.5- to 52.5-fold volume) of iMatrix-511 (MATRIXOME) and PBS/MgCl₂ (25 mL), and the bag was then aseptically connected to Prodigy. iMatrix-511 and PBS/MgCl₂ were poured into the chamber, and the chamber was precoated overnight at a temperature set at 22°C.

### (4) SeV Infection

On the following day, in the safety cabinet, a reagent bag was filled with 8 ml of 2% HSA HSC brew, 0.3 ml of SeV, and 18 ml of six cytokines, and aseptically connected to Prodigy. The bag containing PBMC was connected to Prodigy. The coating solution was removed from the chamber. By setting the conditions using the Activate Shaker type 1 and Deactive Shaker modules of the Activity Matrix program of Prodigy, PBMC (2 ml: 5 × 10⁶ cells) and a SeV-containing culture solution (8 ml) were put into the chamber, shaken (rotated) for one minute every 30 minutes, and allowed to stand for two hours (37°C) to cause infection.

### (5) Culture

After SeV infection, with the Prodigy program (the culture module of the Adherent Cell Culture (ACC) System), 2% HSA HSC Brew (10 mL) was transferred and culture was started (day 0 post-establishment).

On day 5 post-establishment, in the safety cabinet, a Terumo separation bag was filled with 200 mL of StemFit (registered trademark) AK03N (manufactured by Ajinomoto Healthy Supply Co., Inc.) and 200 µL of iMatrix-511. The HSC brew bag was aseptically detached from Prodigy, and the bag containing StemFit was manually connected thereto. With the Prodigy program, 100 mL of StemFit was put into the chamber by feed. With the Prodigy program, 50 mL of the culture medium was automatically discharged, and culture medium replacement was performed by putting 20 mL of StemFit into the chamber. Culture was continued.

On day 7 post-establishment, in the safety cabinet, a Terumo separation bag was manually filled with 200 mL of StemFit (registered trademark) AK03N and 200 µL of iMatrix-511, and the StemFit-containing bag previously connected to Prodigy was manually detached therefrom with a heat sealer and replaced with the new Terumo separation bag by connection using a sterile connecting device. With the Prodigy program, 100 mL of StemFit was inputted by feed, 129 ml of the culture medium was discharged, culture medium replacement was then automatically performed by input of 5 mL of StemFit, and culture was continued. On day 7 or later post-establishment, culture medium replacement was automatically performed with the Prodigy program by partially removing the old culture medium in the chamber by centrifugation and adding a fresh culture medium into the chamber. Culture medium replacement was performed every two days until day 16 post-establishment.

### (6) Harvest

On day 19 post-establishment, 25 ml of TrypLETM Select (1X) mixed with a 0.5 mmol/L EDTA/PBS solution at 1:1 was added into the chamber and allowed to stand at 39°C for 10 minutes, and the cells were released from the chamber. The cells released and collected were seeded in a 48-well plate, a suitable amount of AK03N, a ROCK inhibitor at a final concentration of 10 µM, and 1 µl of iMatrix-511 were added thereto, and the cells were cultured in the 48-well plate. On the following day, the ROCK inhibitor was removed by replacing with AK03N not containing the ROCK inhibitor. On day 25 post-establishment, iPS cell colonies were confirmed using a microscope.

### (7) Results

iPS cell colonies were microscopically observed on day 23 post-establishment, and 6.6 × 10⁴ iPS cells were observed on day 27 post-establishment. Accordingly, it was confirmed that separation of PBMC from whole blood, SeV infection, culture, and establishment of iPS cells could be continuously performed in the closed system within one device. On day 24 post-establishment, the culture medium was replaced with 500 µL/well of AK03N. On day 26 post-establishment, 1.3 × 10⁴ cells/well of the 48-well plate were passaged to one well of a 6-well plate, and all the remaining cells of the wells of the 48-well plate were passaged to one well of the 6-well plate.

### Example 2

### (1) Preliminary Preparation

A tubing set was attached as in Example 1. A leukocyte removal filter was prepared and horizontally placed in a closed state under shading at room temperature. In a safety cabinet, a bag containing 60 mL of HSA was mixed and filled with 3 L of PBS/EDTA buffer, and a bag containing 20 mL of HSA was mixed and filled with saline. Saline was flown into Prodigy with the Prodigy program.

### (2) Separation of CD34-positive Cells from PBMC

Whole blood was filtered through the leukocyte removal filter, and then, in the safety cabinet, the blood remaining in the filter was manually eluted with 50 mL of PBS(-), and PBMC was prepared by Ficoll separation. The obtained 2 × 10⁸ PBMC cells were manually mixed with 200 ml of 0.5% HSA/PBS(-) in the safety cabinet and used as a starting material. The debris was manually removed from PBMC as in Example 1. CD34-positive cells were magnetically removed with the Prodigy program "T cell transduction process" using microbeads previously kept at room temperature. 40 ml of a liquid containing the obtained CD34-positive cells was eluted with the program, and 4 ml out of the liquid containing the CD34-positive cells was aseptically taken into a sampling bag which is a part of the tubing set by holding the bag by hand to draw pressure. The CD34-positive cells taken out were counted with an automatic cell counter to find that the cell count was 1.52 × 10⁵ cells/36 mL. It was decided that the approximately 1.52 × 10⁵ cells would all be subjected to infection, and the amount of Sendai virus vector would be 1.5 ml. The culture medium was replaced with StemSpan-AOF with the program.

The cells were fixed and then the nuclear staining was performed with DAPI, and the percentages of CD45- and CD34-positive cells were examined with FACS. The results are as shown in FIG. 1.

**[Table 1]**

| | DAPI | CD45 | CD34 |
|---|---|---|---|
| Before separation | 96% | 80% | 0.17% |
| After separation | 80% | - | 1% |

1% of the cells were CD34-positive cells, and the cell count was theoretically estimated to be 1.52 × 10³ cells.

### (3) SeV Infection

The chamber was aseptically removed and placed in the safety cabinet, the connected tubing set was switched from TS730 to T520, and the removed chamber was aseptically connected to Prodigy again. In the safety cabinet, a bag was filled with 1 L of StemSpan and aseptically connected to Prodigy. With the program, a liquid containing the approximately 1.52 × 10³ CD34-positive cells obtained was concentrated to a volume of 10 ml in the chamber. With the program, 1.5 mL of SeV 5.1 × 10⁷/mL and 20 mL of StemSpan were put into the chamber, and infection was performed in a total volume of 20 mL in the chamber. Infection was performed while centrifuging at 32°C for two hours using the spin module in the Activity Matrix program of Prodigy. With the program, the culture was washed with 600 mL of StemSpan twice and then reduced to a volume of 10 mL.

### (4) Culture

StemSpan, six cytokines, and iMatrix-511 were simultaneously put into the chamber to prepare a mixture at 1 µg/cm². 200 µL of iMatrix and 180 µL of six cytokines were put into a final volume of 30 mL. 200 mL of StemSpan + 200 µL of iMatrix + 180 µL of six cytokines were inputted by feed to a final volume of 30 mL.
- On day 1 post-establishment: 20 mL of StemFit + 200 µL of iMatrix
- On day 3 post-establishment: 30 mL of StemFit was added to the chamber by feed
- On day 5 post-establishment: 50 mL of StemFit was added to the chamber by feed
- On days 7, 9, 12, 14, 16, 19, 21, and 23 post-establishment: StemFit culture medium was replaced by removing 50 mL and adding 50 mL, such that the volume of the culture in the chamber was 130 mL.

### (5) Harvest

On day 26 post-establishment, the tube connected to the chamber was disconnected and heat-sealed using a tube sealer, such that the chamber was aseptically detached from Prodigy. A lid and a sterile filter were attached to the portion of the chamber to be brought into contact with the outside, and the cells were aseptically observed using a microscope in a state where they were contained in the chamber.

### (6) Results

The results of aseptic observation of the cells using the microscope showed that many spheroids were formed in the chamber. On day 28 post-establishment, the number of spheroids was counted with a counter to be about 308.

One-eighth the volume of the obtained cell culture was collected and dissociated into single cells by 30-minute TrypLE processing, and the cell count was counted. Since the number of single cells in one-eighth the volume of the culture was 1.26 × 10⁶, the number of the obtained iPS cells was 1.0 × 10⁷. Since the number of live single cells in one-eighth the volume of the culture was 1.18 × 10⁶, the number of the obtained live iPS cells was 9.44 × 10⁶, and viability was 94%.

### (7) Differentiation Induction

In accordance with the protocol described in Noriko Shimasaki, et al. Cytotherapy 25 (2023) 1338-1348, differentiation of the obtained iPS cells into monocytes was induced using the Prodigy program (TCT, Empty Matrix), while maintaining the closed system and leaving the scaffold material as is in the Prodigy chamber. Specifically, on the first day of differentiation, AK03N culture medium was removed, and Essential 8 Medium supplemented with 160 ng/mL BMP4, 160 ng/mL VEGF, and 8 µM CHIR was added to the chamber. Two days later, the culture medium was replaced with Essential 6 Medium (Gibco) supplemented with 160 ng/mL VEGF, 100 ng/mL SCF, 100 ng/mL FGF-2 (Miltenyi Biotec, 130-093-841), and 4 µM SB431542 (1614/10, TOCRIS). Replacement with the fresh culture medium was performed while centrifuging. Four days after the start of differentiation, the culture medium was replaced with StemPro-34 culture medium (Gibco, 10640-019) containing 80 ng/mL VEGF, 100 ng/mL SCF (Miltenyi Biotec), 20 ng/mL Tpo (288-TPN-025, R&D Systems), 100 ng/mL IL-3 (203IL-050, R&D Systems), and 100 ng/mL FLT-3 (308-FK-025/CF, R&D Systems). Three days later, the culture medium was replaced with the same culture medium containing 100 ng/mL M-CSF (316-MC-100, R&D) instead of VEGF. Ten days after the start of differentiation, the culture medium was replaced with StemPro-34 culture medium supplemented with 100 ng/mL FL, 100 ng/mL M-CSF, and 50 ng/mL GM-CSF (215-GM-050, R&D). Equal volumes of fresh culture media were added every two days. As a result, monocytes were observed on day 10 after the start of differentiation induction. Accordingly, it could be confirmed that monocytes were obtained by performing a series of steps of isolation of PBMC from whole blood, SeV infection (spin infection), iPSC establishment, and differentiation into monocytes, while continuously maintaining the closed system in the same chamber in Prodigy.

### (8) Evaluation of Differentiation into Monocytes by Flow Cytometry

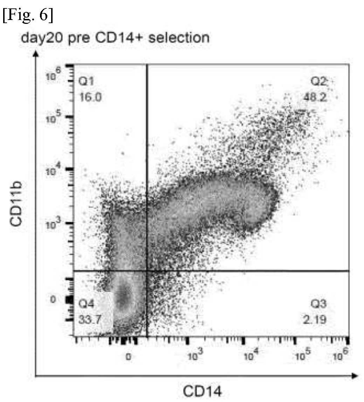

On the final day of differentiation, the cells were released from the chamber using TrypLE Select and then counted by staining with trypan blue. The proportion of CD14-positive cells was analyzed through flow cytometry. The results of expression of monocyte differentiation markers, CD14- and CD45-positive cells, are shown in FIG. 6. The proportion of differentiated monocytes was 48.2% relative to all the live cells.

### Example 3

### (1) Preliminary Preparation

Preliminary Preparation was performed as in Example 2, except that T520 was assembled as a tubing set.

### (2) Precoating of Inside of Chamber with Scaffold Material

In a safety cabinet, a chamber was manually precoated with an acrylic resin containing a peptide having an RGD sequence. 0.5 µg/µL each of PBS and iMatrix-511 were mixed to prepare 1 µg/cm² iMatrix 511/100 µL. iMatrix-511 was returned to room temperature. 200 µL of iMatrix-511 and 200 µL of iMatrix-332 in 40 mL were prepared. The coating volume was set to 25 mL, the coating temperature was set to 22°C, and the coating time was set to 24h with the Prodigy program, the chamber was automatically coated, and then 45 mL of the coating solution was removed.

### (3) Preparation of Starting Material

In the safety cabinet, a leukocyte removal filter was manually washed with PBS, and PBMC was removed from the obtained liquid containing blood using Ficoll. The cells were counted, and 1 × 10⁸ cells were selected for SeV infection. In the safety cabinet, a bag was aseptically filled with the obtained PBMC together with 100 mL of StemSpan manually. The bag was aseptically connected to Prodigy. It was replaced with StemSpan with the Prodigy program. With the program, PBMC was concentrated to a volume of 10 mL in the chamber.

### (4) SeV Infection

SeV infection was performed as in Example 1, except that 1 × 10⁸ cells were subjected to infection, 1.5 mL of SeV was used, and the total volume in the chamber was 20 mL.

### (5) Culture

The volume of the culture was reduced to 10 mL by performing volume reduction. Preparation for the start of culture was performed as in Example 1. The following culture steps were inputted from the screen of Prodigy, and culture was automatically performed with the program of Prodigy.
Day 0 post-establishment: 20 mL of StemSpan + 180 µL of six cytokines + 200 µL of iMatrix-511 + 200 µL of iMatrix-332) 400 µL (iMatrix 1 µg/cm²), final volume: 30 ml
Day 1 post-establishment: 20 mL of StemFit AK03N + 200 µL of iMatrix-511 + 200 µL of imatrix-332
Day 3 post-establishment: 30 mL of StemFit AK03N was added to the chamber by feed
Day 6 post-establishment: 50 mL of StemFit AK03N was added to the chamber by feed
On day 7 or later post-establishment, culture medium replacement was performed by partially removing the old culture medium by centrifugation and adding a fresh culture medium in the chamber. Culture medium replacement was performed every two or three days (the volume of the chamber was 30 to 130 mL).
Culture was performed in Prodigy since the establishment until day 13 post-establishment, the chamber was aseptically removed from Prodigy on day 14 post-establishment, and culture was continued in a 5% CO₂ incubator.

### (6) Harvest

The cells were not released from the chamber, the chamber itself was aseptically removed from Prodigy, and the cells were observed using a microscope.

### (7) Results

Microscopic observation on day 14 post-establishment showed that almost no spheroids and many adherent colonies were formed in the chamber. On day 28 post-establishment, the number of colonies was about 284, and the number of live cells was 4.88 × 10⁷, as counted with a counter.

### Example 5

Example 5 was performed as in Example 3, except that twice the amount of the scaffold material used in Example 3 was used for precoating, and no other additional coating was performed. As a result, microscopic observation on day 18 post-establishment showed that adherent colonies were formed in the chamber. The number of colonies was about 482 on day 21 post-establishment, and the number of live cells was 6.4 × 10⁷ on day 28 post-establishment, as counted with a counter.

### Example 7

### (1) Preliminary Preparation

Preliminary Preparation was performed as in Example 1, except that, in the safety cabinet on the previous day, tubing sets TS730 and TS520 were checked and tightened, and 150 mL of Ficoll, 0.5% HSA PBS/EDTA: 60 mL of Albuminar/PBS/3 L of EDTA, and 0.5% HSA/saline: 20 mL of Albuminar/1 L of saline were prepared as buffers or the like.

### (2) Preparation of Starting Material

Separation was performed as in Example 1, except that whole blood from a volunteer donor was filtered through a leukocyte removal filter, the blood remaining in the filter (filtered blood) was then eluted with 50 mL of PBS (-) and prepared as a starting material, and 500 mL of 0.5% HSA/saline was used.

### (3) SeV Infection

The same chamber as in the step of separating PBMC from whole blood was used, and the connected tubing set was switched from TS730 to TS520. The chamber was washed with 0.5% HSA PBS/EDTA. 1 × 10⁸ cells were subjected to infection, 1.5 mL of SeV (MOI 5.1 × 10⁷/mL) was used, the SeV was diluted with 10 ml of StemSpan, and then the total volume was brought to 20 mL. The "T cell culture 3" program was started. SeV infection was performed as in Example 1 except for the above-mentioned operations. 20 mL of StemSpan was inputted by feed. Concentration brought the final volume in the chamber to 10 mL.

### (4) Culture

Culture was performed as in Example 2, except that iMatrix-511 was added at 200 µL instead of 1 mL when cells were seeded on day 0 post-establishment, and a scaffold material was not added on day 1 post-establishment. On days 7, 9, and 11 post-establishment, culture medium replacement was automatically performed with the Prodigy program. On day 15 or later post-establishment, culture medium replacement was performed manually.

### (5) Harvest

The cells were not released from the chamber, the chamber itself was aseptically removed from Prodigy on day 15 post-establishment, and the cells were observed using a microscope.

### (6) Results

Microscopic observation on day 29 post-establishment showed that spheroids were formed in the chamber. On day 29 post-establishment, the number of spheroids was about 86, and the number of live cells was 6.98 × 10⁶, as counted with a counter.

### Example 8

Separation of PBMC from filtered blood, SeV infection, and culture were performed as in Example 7, except that filtered blood from the same donor as in Example 7 was used, and 200 µL of iMatrix was added on day 1 post-establishment. As a result, microscopic observation on day 29 post-establishment showed that spheroids were formed in the chamber. On day 29 post-establishment, the number of spheroids was about 28, and the number of live cells was 1.09 × 10⁶, as counted with a counter.

### Example 9

### (1) Separation of PBMC from Whole Blood, SeV Infection, and Culture

Preliminary preparation was performed as in Example 7. Separation of PBMC from whole blood was performed as in Example 1, except that 40 ml of peripheral blood (whole blood) from a volunteer donor and 40 mL of PBS were used as starting materials. SeV infection was performed as in Example 7. Culture was performed as in Example 1, except that iMatrix-511 was added at 2 mL instead of 1 mL when cells were seeded on day 0 post-establishment, and a scaffold material was not added on day 1 post-establishment. The cells were harvested from the chamber without release.

### (2) Results

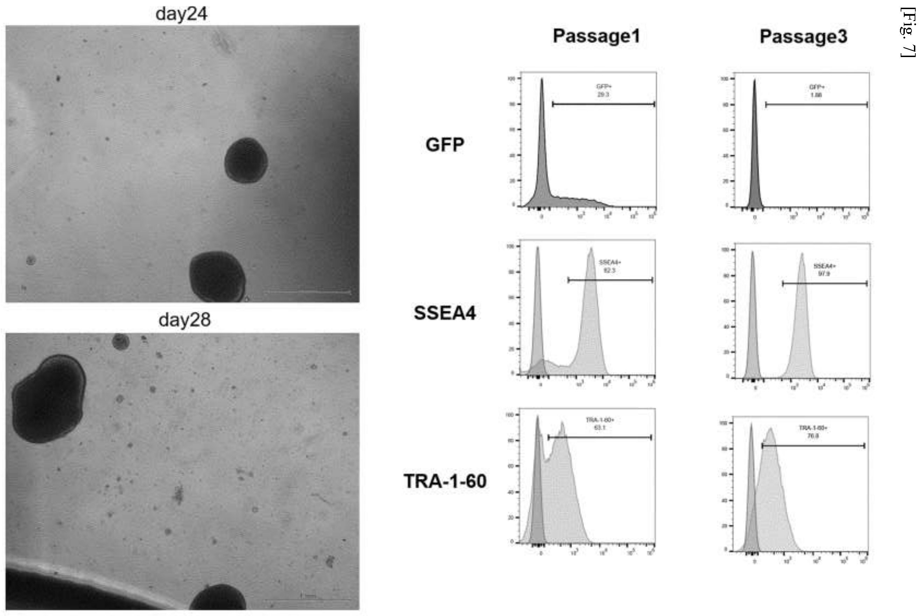

Microscopic observation on day 28 post-establishment showed that spheroids were formed in the chamber. Photographs of the spheroids on day 24 (FIG. 7) and day 28 (FIG. 7) post-establishment are shown. On day 28 post-establishment, the number of spheroids was about 424, and the number of live cells was 4.21 × 10⁶, as counted with a counter. On day 28 post-establishment, 2 mL of iMatrix332 was added and then partially adhered. On day 31 post-establishment, 3 mL of iMatrix332 was added but was not adhered anymore. The adhesion ratio was about 30% based on visual observation.

### (3) Passage

Passage was performed on day 28 post-establishment.

### (4) Flow Cytometry

Expression of GFP carried in a vector and representative iPS cell markers, SSEA4 and TRA-1-60, at passages 1 and 3 was confirmed. The results are shown in FIG. 7. SSEA4 and TRA-1-60 were confirmed by immunostaining with an antibody. These results confirmed that iPS cells could be established.

### Example 10

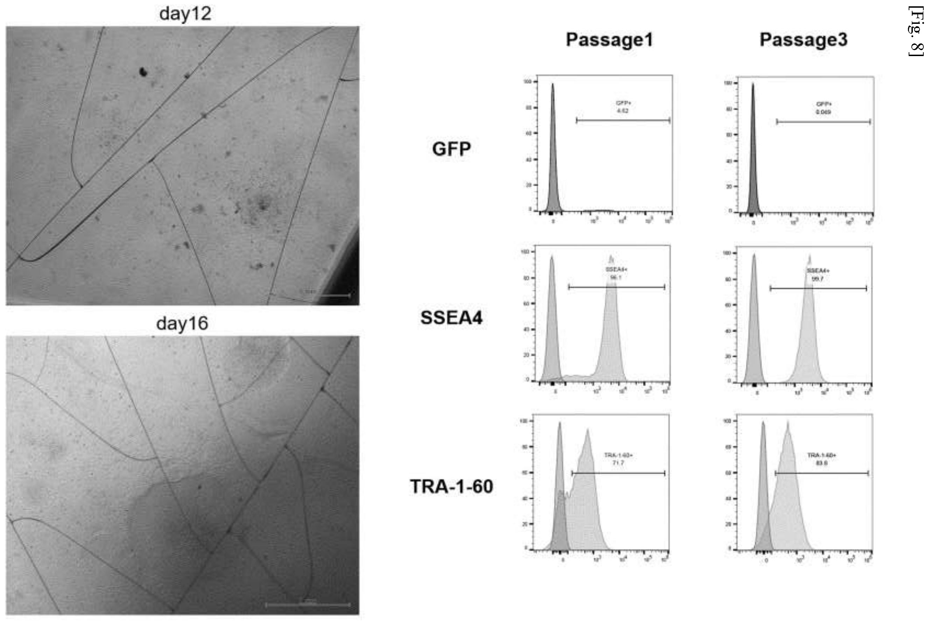

Preliminary preparation was performed as in Example 7. Precoating of the inside of a chamber with a scaffold material was performed as in Example 5, preparation of a starting material (separation of PBMC from whole blood) was performed as in Example 9, SeV infection was performed as in Example 7, culture was performed as in Example 5, and harvest was performed as in Example 7. As a result, microscopic observation on day 21 post-establishment showed colonies in the chamber. On day 21 post-establishment, the number of colonies was about 402, and the number of live cells was 5.48 × 10⁷, as counted with a counter. Photographs of the cells on days 12 and 16 post-establishment are shown in FIG. 8.

### (2) Passage

Passage was performed on day 21 post-establishment.

### (3) Flow Cytometry

Expression of GFP carried in a vector and SSEA4 and TRA-1-60 at passages 1 and 3 was confirmed one week after each passage. The results are shown in FIG. 8. These results confirmed that iPS cells could be established.

### Example 12

### (1) Separation of PBMC from Whole Blood, SeV Infection, and Culture

Separation of PBMC from whole blood, SeV infection, and culture were performed as in Example 9, except that 1 ml of iMatrix-332 was used as a scaffold material during culture. As a result, microscopic observation on day 28 post-establishment showed that spheroids were formed in the chamber. On day 28 post-establishment, the number of spheroids was about 555 (some of which form colonies), and the number of live cells was 2.59 × 10⁶, as counted with a counter.

### Example 13

### (1) Separation of PBMC from Whole Blood, SeV Infection, and Culture

Separation of PBMC from whole blood, SeV infection, culture, and harvest were performed as in Example 12, except that the volumes of starting materials, whole blood and PBS, were 20 mL and 20 mL, respectively, and the scaffold material iMatrix-322 was added at 2 mL instead of 1 mL.

### (2) Results

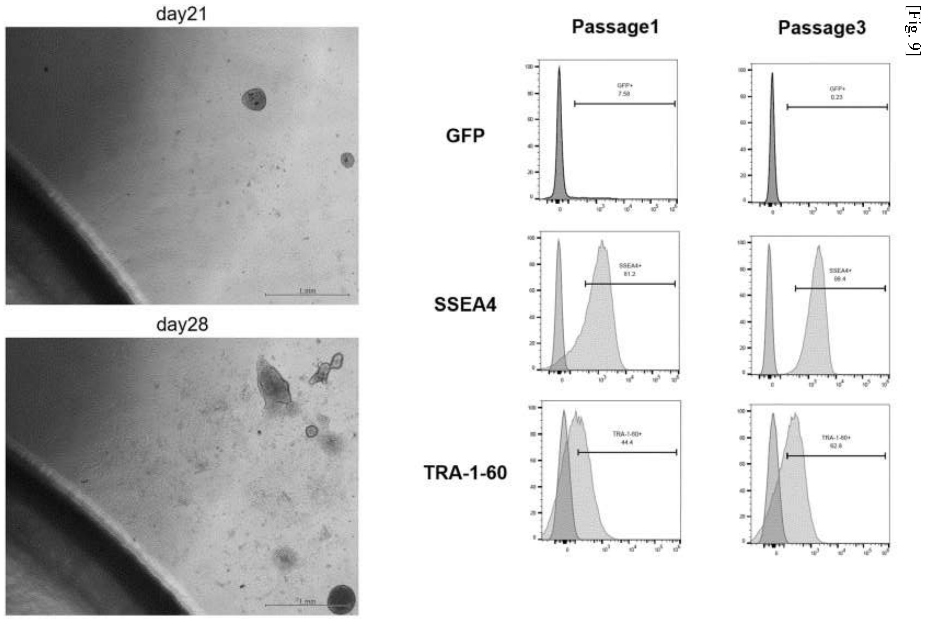

Microscopic observation on day 28 post-establishment showed that spheroids were formed in the chamber. On day 28 post-establishment, the number of spheroids was about 518 (some of which form colonies), and the number of live cells was 7.46 × 10⁶, as counted with a counter. Photographs of the cells on days 12 and 16 post-establishment are shown in FIG. 9.

### (3) Passage

Passage was performed on day 21 post-establishment.

### (4) Flow Cytometry

Expression of GFP carried in a vector and SSEA4 and TRA-1-60 at passages 1 and 3 was confirmed one week after each passage. The results are shown in FIG. 9. These results confirmed that iPS cells could be established.

### Example 14

### (1) Separation of PBMC from Whole Blood, SeV Infection, and Culture

Preliminary preparation was performed as in Example 9, precoating of the inside of a chamber with a scaffold material was performed as in Example 5, preparation of a starting material (separation of PBMC from whole blood) and SeV infection were performed as in Example 13, and culture was performed as in Example 10. The cells were not released from the chamber, and the obtained harvest was directly subjected to differentiation induction.

### (2) Differentiation Induction

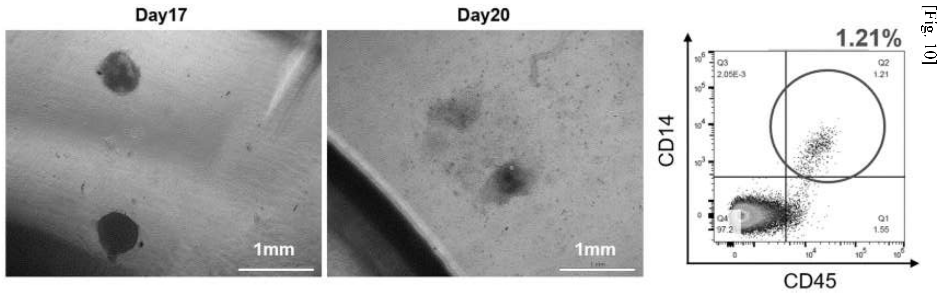

On day 21 or later post-establishment, differentiation induction was performed by the same method as in Example 2. Differentiation of iPS cells was induced until day 17 post-establishment. The cells were aseptically observed using a microscope in a state where they were contained in the chamber. As a result, monocytes were observed as shown in FIG. 10 on days 17 and 20 after the start of differentiation induction. Accordingly, it could be confirmed that monocytes were obtained by performing a series of steps of isolation of PBMC from whole blood, SeV infection, iPSC establishment, and differentiation into monocytes, while continuously maintaining the closed system in the same chamber in Prodigy.

### (3) Evaluation of Differentiation into Monocytes by Flow Cytometry

Differentiation was evaluated by flow cytometry as in Example 2. The results of expression of monocyte differentiation markers, CD14- and CD45-positive cells, are shown in FIG. 10. The proportion of differentiated monocytes was 1.21% relative to all the live cells.

### (4) Differentiation of Monocytes into Macrophages

On day 20 after the start of differentiation induction, the cells were seeded in a 6-well plate and cultured in TexMACS medium (Miltenyi Biotec) containing 50 ng/mL M-CSF for five to seven days to induce differentiation of monocytes into macrophages. FACS was performed to find that the percentage of M0 macrophage markers, CD14-positive cells, in the live cells was 1.21% on day 5 and 15.4% on day 7 after the start of culture following stimulation with M-CSF.

### (5) Confirmation of Phagocytotic Activity of Macrophages

The cells were seeded in a 96-well plate. Red fluorescent latex beads having an average diameter of 2 µm (L3030; Sigma-Aldrich) was diluted with 50 µL of TexMACS medium, and added to the cells at a final concentration of 5 µL/mL. The cells were incubated at 37°C for four hours and then collected. The collected cells were washed with Cell Staining Buffer and labeled with an APC-conjugated anti-CD14 antibody (BD Pharmingen, San Diego, CA, USA). The cells were analyzed by flow cytometry to confirm the percentage of cells incorporating L3030 in the CD14-positive cells, which reflects non-specific phagocytotic activity. Thus, the presence of phagocytotic activity was confirmed.

### Example 15

### (1) Separation of PBMC from Whole Blood, SeV Infection, and Culture

Separation of PBMC from whole blood, SeV infection, culture, and harvest were performed as in Example 13, except that 5 × 10⁷ cells were subjected to SeV infection, spin infection was used as in Example 2, and the scaffold material during cell seeding was 5 mL of iMatrix-332. As a result, microscopic observation on day 28 post-establishment showed that colonies and spheroids were formed in the chamber.

### Example 16

As preliminary preparation, the inside of a chamber was precoated with a scaffold material. Specifically, in a safety cabinet, a bag was aseptically filled with five vials of Synthemax and sealed, and the bag was aseptically connected to Prodigy via a tube. The scaffold material was injected into the chamber with the Prodigy program (the coating module of Adherent Cell Culture (ACC) System) and used for precoating overnight. Separation of PBMC from whole blood, SeV infection, culture, and harvest were performed as in Example 15, except that the scaffold material during cell seeding, iMatrix-332, was not added. As a result, microscopic observation on day 21 post-establishment showed that colonies were formed in the chamber. On day 21 post-establishment, the number of colonies was about 202, and the number of live cells was 1.74 × 10⁷, as counted with a counter.

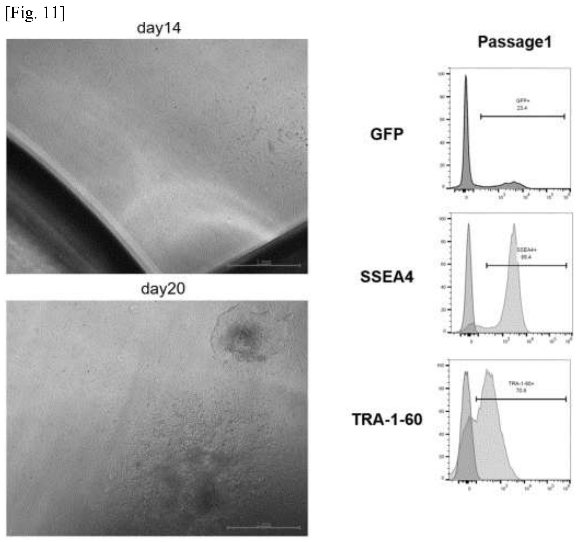

Photographs of the cells on days 14 and 20 post-establishment are shown in FIG. 11. Passage was performed on day 21 post-establishment. Expression of GFP carried in a vector and SSEA4 and TRA-1-60 at passage 1 was confirmed one week after the passage. The results are shown in FIG. 11. These results confirmed that iPS cells could be established.

### Example 17

Example 17 was performed as in Example 16, except that, after separating PBMC from whole blood, the connected tubing set including a chamber was switched to TS520, and three vials of Synthemax were injected into the chamber and used for precoating at RT for 2 hr. As a result, microscopic observation on day 21 post-establishment showed that colonies were formed in the chamber. On day 21 post-establishment, the number of colonies was about 297, and the number of live cells was 1.18 × 10⁷, as counted with a counter.

### Example 18

Example 18 was performed as in Example 17, except that the coating time was 30 minutes. Microscopic observation on day 21 post-establishment showed that colonies were formed in the chamber. On day 21 post-establishment, the number of colonies was about 189, and the number of live cells was 1.37 × 10⁷, as counted with a counter.

### Example 19

Example 19 was performed by the same method as in Example 17, except that precoating after Ficoll separation was not performed, a TS520 chamber was coated with three vials of Synthemax at 22°C overnight, and then 5 × 10⁷ cells were moved to the chamber and subjected to SeV infection. As a result, iPS cell colonies were observed. The experiment was performed twice and produced the same results.

### Example 22

Example 22 was performed by the same method as in Example 17, except that coating was performed with six vials of 500 µg of Laminin 521 at 6°C overnight. As a result, iPS cell colonies were observed. The results in the Examples are summarized in Tables 2 to 4 below.

**[Table 2]**

| | Example 1 | Example 2 | Example 3 | Example 5 | Example 7 | Example 8 |
|---|---|---|---|---|---|---|
| Starting material | 70 mL of whole blood | PBMC | PBMC | PBMC | Filtered blood | Filtered blood |
| Coating | 1 mL of iMatrix511 mixed and seeded 200 µL of iMatrix added on the following day | 200 µL of iMatrix511 mixed and seeded 200 µL of iMat added on the following day | RGD sequence coating + 200 µL of iMat511 + 200 µL of iMat332 mixed and seeded + 200 µL of iMat511 + 200 µL of iMat332 added on the following day | RGD sequence coating, twice amount | 200 µL of iMatrix511 mixed and seeded | 200 µL of iMatrix511 mixed and seeded + 200 µL of iMat added on the following day |
| Method for separation from whole blood | Ficoll separation | Magnetic separation (CD34) | Ficoll separation | Ficoll separation | Ficoll separation | Ficoll separation |
| Infection method | Allowing to stand for 2 hr (shaking for 1 min every 30 min) | Spinning for 2 hr | Allowing to stand for 2 hr (shaking for 1 min every 30 min) | Allowing to stand for 2 hr (shaking for 1 min every 30 min) | Allowing to stand for 2 hr (shaking for 1 min every 30 min) | Allowing to stand for 2 hr (shaking for 1 min every 30 min) |
| | 5 x 10⁶ cells used | 1.52 x 10⁵cells used | 1 x 10⁸ cells used | 1 x 10⁸ cells used | 1 x 10⁸ cells used (the same donor as in Example 8) | 1 x 10⁸ cells used(the same donor as in Example 7) |
| iPSC establishment | Released and seeded in 48wp with Prodigy | About 308 spheroids and 9.44 x 10⁶ live cells on day 28 post-establishment | About 284 colonies and 4.88 x 10⁷ live cells on day 28 post-establishment | Colonies could be confirmed on day 18 post-establishment | About 86 spheroids and 6.98 x 10⁶ live cells on day 29 post-establishment | About 28 spheroids and 1.09 x 10⁶ live cells on day 29 post-establishment |
| | Colonies confirmed on day 25 (P1) post-establishment 6.6 x 10⁴ cells on day 27 | | | About 482 colonies on day 21 post-establishment 6.4 x 10⁷ live cells on day 28 post-establishment | | |
| Differentiation induction | Not performed | iPSC differentiated into monocytes | Not performed | Not performed | Not performed | Not performed |

**[Table 3]**

| | Example 9 | Example 10 | Example 12 | Example 13 | Example 14 | Example 15 |
|---|---|---|---|---|---|---|
| Starting material | 40 mL of whole blood | 40 mL of whole blood | 40 mL of whole blood | 20 mL of whole blood | 20 mL of whole blood | 20 mL of whole blood |
| Coating | 2 mL of iMat511 mixed and seeded | RDG sequence coating, twice the amount used in Example 3 | 1 mL of iMat332 mixed and seeded | 2 mL of iMat332 mixed and seeded | RDG sequence coating, twice the amount used in Example 3 | 5 mL of iMat332 mixed and seeded |
| Method for separation from whole blood | Ficoll separation | Ficoll separation | Ficoll separation | Ficoll separation | Ficoll separation | Ficoll separation |
| Infection method | Allowing to stand for 2 hr (shaking for 1 min every 30 min) | Allowing to stand for 2 hr (shaking for 1 min every 30 min) | Allowing to stand for 2 hr (shaking for 1 min every 30 min) | Allowing to stand for 2 hr (shaking for 1 min every 30 min) | Allowing to stand for 2 hr (shaking for 1 min every 30 min) | Spinning for 2 hr 5 x 10⁷ cells used |
| | 1 x 10⁸ cells used | 1 x 10⁸ cells used | 1 x 10⁸ cells used | 5 x 10⁷ cells used | 5 x 10⁷ cells used | |
| iPSC establishment | About 424 spheroids and 4.21 x 10⁶ live cells on day 28 post-establishment | About 402 colonies and 5.48 x 10⁷ live cells on day 21 post-establishment | About 555 spheroids (some of which formed colonies) and 2.59 x 10⁶ live cells on day 28 post-establishment | About 518 spheroids (some of which formed colonies) and 7.46 x 10⁶ live cells on day 28 post-establishment | Monocyte differentiation started on day 21 post-establishment | Small colonies confirmed on day 15 post-establishment |
| | | | | | | Colonies and spheroids coexisted on day 20 post-establishment |
| | | | | | | Colonies or spheroids on day 24 post-establishment |
| Differentiation induction | Not performed | Not performed | Not performed | Not performed | iPSC differentiated into monocytes and then into macrophages | Not performed |

**[Table 4]**

| | Example 16 | Example 17 | Example 18 |
|---|---|---|---|
| Starting material | 20 mL of whole blood | 20 mL of whole blood | 20 mL of whole blood |
| Coating | 5 vials of Synthemax | 3 vials of Synthemax | 3 vials of Synthemax |
| | Precoating after Ficoll | Precoating after Ficoll (TS520 chamber, RT, 2 hr) | Precoating after Ficoll (TS520 chamber, 37°C, 30 min) |
| Method for separation from whole blood | Ficoll separation | Ficoll separation | Ficoll separation |
| Infection method | Spinning for 2 hr | Allowing to stand for 2 hr (shaking for 1 min every 30 min) | Allowing to stand for 2 hr (shaking for 1 min every 30 min) |
| | 5 x 10⁷ cells used | | |
| | | 5 x 10⁷ cells used | 1 x 10⁷ cells used |
| iPSC establishment | About 202 colonies and 1.74 x 10⁷ live cells on day 21 post-establishment | About 297 colonies and 1.18 x 10⁷ live cells on day 21 post-establishment | About 74 colonies on day 21 post-establishment |
| | | | About 189 colonies and 1.37 x 10⁷ live cells on day 28 post-establishment |

### Examples 23 and 24

Methods for coating with a scaffold material were examined by establishing iPS cells by the same method as in Example 13 except for the conditions shown in Table 5 below.

**[Table 5]**

| | Example 23 | Example 24 |
|---|---|---|
| Amount of iMatrix-511 | 20 times the typical amount | 5 times the typical amount |
| Coating method | Mixing and applying during cell seeding | Applying before cell seeding |
| Day of confirmation of establishment (days after SeV infection) | Day 24 | Day 21 |
| Results | FIG. 13 | FIG. 13 |
| iPSC shape | 3D (spheroids) | 2D (colonies) |

The examination results showed that the iPS cells that could be established did not have a two-dimensional shape but had a three-dimensional shape (were spheroids) when a scaffold material was mixed with cells and applied during cell seeding. The iPS cells that could be established had a two-dimensional shape (were colonies) when a scaffold material was applied before cell seeding. It was also suggested that a scaffold material is preferably applied before cell seeding in terms of the amount of the scaffold material used (FIG. 13).

### Examples 25 to 27

Sev amounts were examined by establishing iPS cells by the same method as in Example 13 except for the conditions shown in Table 6 below.

**[Table 6]**

| | Example 25 | Example 26 | Example 27 |
|---|---|---|---|
| Amount of SRV4 [µl] | 500 | 200 | 100 |
| Days after SeV infection | Day 21 | Day 20 | Day 27 |
| Number of infected cells | 2.0 x 10⁷ | 5.0 x 10⁷ | 5.0 x 10⁷ |
| Number of cells after establishment | 2.35 x 10⁷ | 1.22 x 10⁷ | 1.14 x 10⁷ |
| Results | FIG. 14 | FIG. 14 | FIG. 14 |

The examination results showed that even the SeV amounts shown in Table 6 can establish iPS cells sufficiently in the production method according to the present invention (FIG. 14).

### Examples 28 to 30 (Summary)

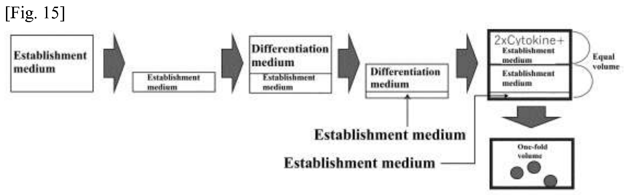

A monocyte differentiation process using a cell processing device (Prodigy) was examined. (1) Culture media and cytokines are as shown in Tables 7 to 10. Although manual operation can remove a medium entirely and replace it with a fresh differentiation medium, a medium cannot be removed entirely according to the specification of the cell processing device (Prodigy). Thus, the old culture medium was diluted with a large volume of a base culture medium, then the liquid volume was reduced by centrifugation, then an equal volume of a differentiation medium containing a two-fold amount of cytokines was added thereto, and then the volume was adjusted such that the medium in the chamber containing cells finally become a one-fold volume of the differentiation medium, thereby completing replacement of the establishment medium with the differentiation medium (FIG. 15).

**[Table 7]**

| | | | | | |
|---|---|---|---|---|---|
| Day | 0 | 1 | 2 | 3 | 4 |
| Cell type | iPSC | Mesodermal | | Hematopoietic endothelial cells | |
| Base culture medium | Essential 8 | | | Essential 6 | |
| Cytokines | BMP4, VEGF, CHIR | | | SB, VEGF, SCF, bFGF | |

**[Table 8]**

| | | | |
|---|---|---|---|
| Day | 5 | 6 | 7 |
| Cell type | Hematopoietic stem and progenitor cells | | |
| Base culture medium | StemPro34 | | |
| Cytokines | VEGF, SCF, Tpo, IL-3, Flt3L | | |

**[Table 9]**

| | | | |
|---|---|---|---|
| Day | 8 | 9 | 10 |
| Cell type | Monocyte progenitor cells | | |
| Base culture medium | StemPro34 | | |
| Cytokines | M-CSF, SCF, Tpo, IL-3, Flt3L | | |

**[Table 10]**

| | | | | | | | | | | |
|---|---|---|---|---|---|---|---|---|---|---|
| Day | 11 | 12 | 13 | 14 | 15 | 16 | 17 | 18 | 19 | 20 |
| Cell type | Monocytes | | | | | | | | | |
| Base culture medium | StemPro34 | | | | | | | | | |
| Cytokines | M-CSF, Flt3L, GM-CSF | | | | | | | | | |

### (2) The process is as follows.

### <Day 0>

(i) 30 ml of AK03N

### <Feed>

(ii) 200 mL of Essential 8 was added (total: 130 mL).
(iii) Dilution of AK03N
(iv) The volume was reduced to 10 mL (AK03N was contained at 3/23 of the 10 mL).
(v) This operation was repeated about twice to dilute AK03N as much as possible.
(vi) The volume was reduced to 10 mL.
(vii) The volume of Essential 8 was determined to be 10 mL.

### <Feed>

(viii) 10 mL of Essential 8 containing 2 x BMP4, 2 x VEGF, and 2 x CHIR was added thereto.
(ix) This was mixed with 10 mL of the Essential 8 in the chamber, such that cells were immersed in Essential 8 culture medium containing 1 x BMP4, 1 x VEGF, and 1 x CHIR.
(x) Differentiation into monocytes in a cell processing device (Prodigy) (similar to the differentiation induction protocol in Example 2)

### Example 28

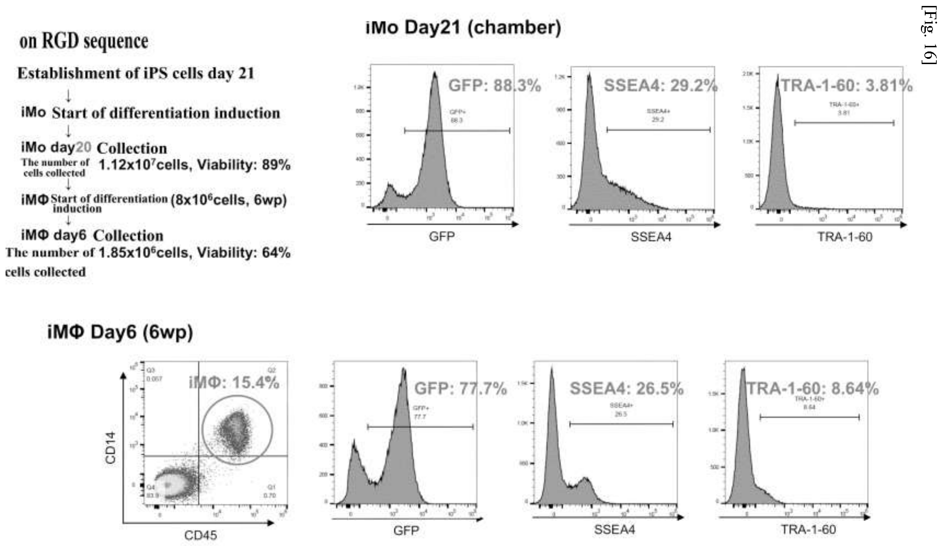

Twice the typical amount of an RGD sequence coating scaffold material was applied to a chamber of a cell processing device (a Prodigy chamber). After infection with Sendai virus vector, on day 21, induction of differentiation into monocytes was started utilizing a mechanism of the cell processing device (Prodigy) in the chamber without removing the chamber from the cell processing device (Prodigy). On day 20 after the start of induction of differentiation into monocytes, the cells (cell count: 1.12 x 10⁷ cells, viability: 89%) were collected. The FACS results were GFP: 88.3%, SSEA4: 29.2%, and TRA-1-60: 3.81% (FIG. 16).

Induction of differentiation into macrophages was started at a cell count of 8 x 10⁶ cells in a 6-well plate, and the cells were collected on day 6 from the start of differentiation induction to find that the cell count was 1.85 x 10⁶ cells and viability was 64%. The FACS results were GFP: 77.7%, SSEA4: 26.5%, and TRA-1-60: 8.64% (FIG. 16). The percentage of M0 macrophage markers, CD14-positive cells, in the live cells was 15.4% on day 6 after the start of culture in the 6-well plate following stimulation with M-CSF.

### Example 29

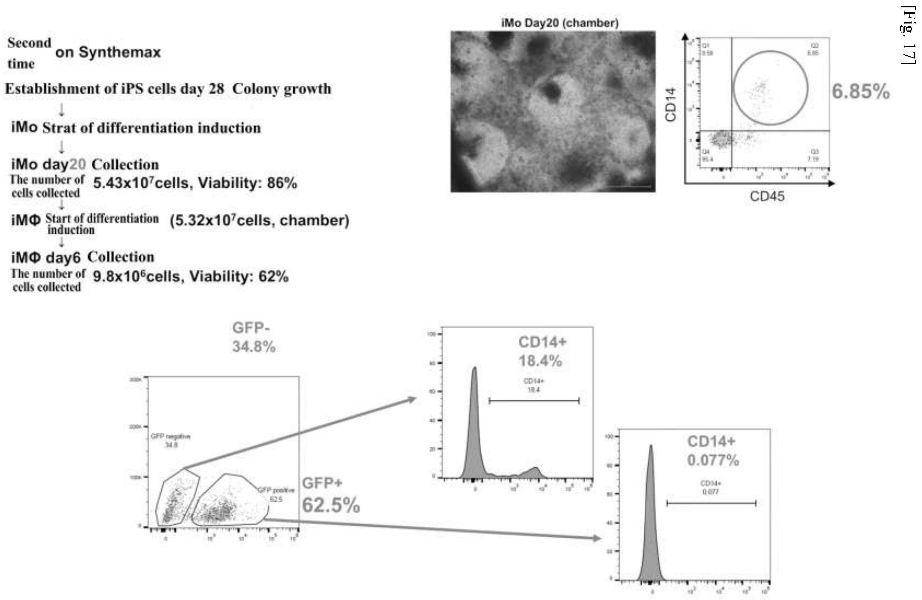

Synthemax was applied to a chamber of a cell processing device (a Prodigy chamber) as a scaffold material. After infection with Sendai virus vector, on day 28, induction of differentiation into monocytes was started in the chamber utilizing a mechanism of the cell processing device (Prodigy) in the chamber without removing the chamber from the cell processing device (Prodigy). On day 20 after the start of induction of differentiation into monocytes, the cells (cell count: 5.43 x 10⁷ cells, viability: 86%) were collected. The results of expression of monocyte differentiation markers, CD14- and CD45-positive cells, showed that the percentage of differentiated monocytes in all the live cells was 6.85% (FIG. 17). GFP analysis by FACS showed that the GFP-positive cells at 62.5%, in which a reprogramming factor remained, were differentiated into extremely few CD14-positive cells at 0.077% and rare monocytes (FIG. 17). Meanwhile, it was found that the GFP-negative cells at 34.8%, in which a reprogramming factor did not remain, were differentiated into CD14-positive cells at 18.4% and monocytes, and the results were considered to be reasonable (FIG. 17). These results suggested that the timing of start of induction is important.

### Example 30

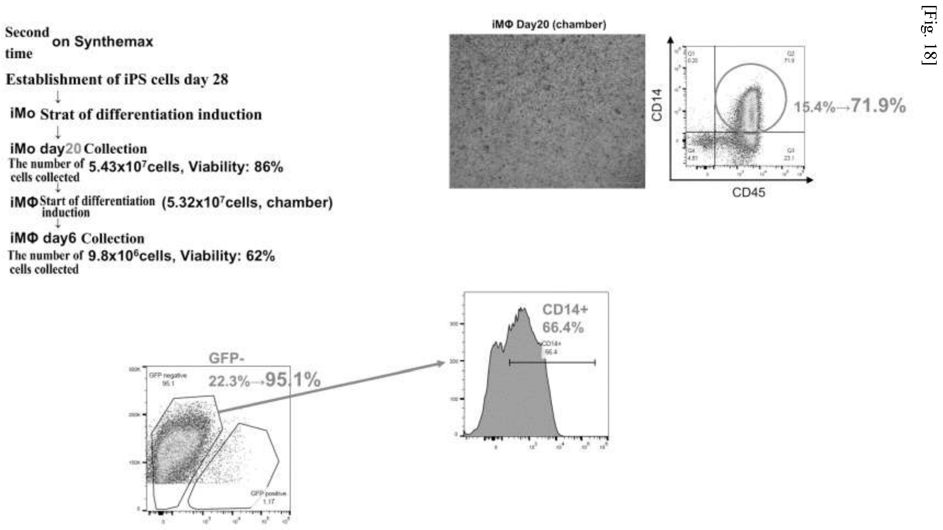

The cells of Example 29 were seeded again in the chamber used in Example 29, and differentiated into macrophages. The results of expression of monocyte differentiation markers, CD14- and CD45-positive cells, showed that the percentage of differentiated macrophages in all the live cells was 71.9% (FIG. 18).

Induction of differentiation into macrophages was started at a cell count of 5.32 x 10⁷ cells in the chamber, and the cells were collected on day 6 to find that the cell count was 9.8 x 10⁶ cells and viability was 62%. GFP analysis by FACS showed that the GFP-positive cells, in which a reprogramming factor remained, were extremely few at 1.17%, while the GFP-negative cells, in which a reprogramming factor did not remain, accounted for 95.1% and were differentiated into CD14-positive cells at 66.4% and macrophages (FIG. 18).

### Industrial Applicability

The production method according to the present invention can continuously perform steps from separation of blood cells from whole blood by centrifugation to establishment of iPS cells using an automated cell processing device in one sealed chamber within an identical closed system portion. This can reduce the cost to produce iPS cells.

The present application claims the benefit of priority based on Japanese Patent Application No. 2023-204958 (filing date: December 4, 2023) filed in Japan, which is incorporated herein by reference in their entirety.

### Reference Numerals

- 100: Sealed chamber
- 210 to 250: Sealed material supply container
- A10: Connecting conduit
- V10: Pinch valve
- F10: Peristaltic pump

## Claims

1. A method for producing induced pluripotent stem cells using a cell processing device,
the cell processing device including a closed system portion in which a sealed material supply container and a sealed chamber rotatable for centrifugation are connected via a connecting conduit,
the production method comprising:
a step (s1) of separating blood cells from whole blood by centrifugation;
a step (s2) of bringing the blood cells into contact with a reprogramming factor; and
a step (s3) of establishing induced pluripotent stem cells from the blood cells, which are performed in the stated order in the sealed rotatable chamber while maintaining closedness of the closed system portion.

2. The method for producing induced pluripotent stem cells according to claim 1, wherein the sealed chamber includes an inlet port and an outlet port and is configured to perform material inflow and outflow via the inlet port and the outlet port in a rotated state for centrifugation or in a state where the rotation is stopped, and
in the step (s1), a fraction containing the blood cells separated by centrifugation remains in the sealed chamber, and another fraction is discharged from the sealed chamber.

3. The method for producing induced pluripotent stem cells according to claim 2, wherein the cell processing device further includes, as a part of a configuration of the closed system portion, a magnetic separation column connected to the sealed chamber via the connecting conduit, and
in the step (s1),
after the centrifugation, the fraction containing the blood cells remains in the sealed chamber,
lymphoid cells among the blood cells are further labeled with magnetic beads, sent to the magnetic separation column, and magnetically separated from the other blood cells, and
the lymphoid cells labeled with the magnetic beads are returned to the sealed chamber.

4. The method for producing induced pluripotent stem cells according to any one of claims 1 to 3, wherein the step (s2) is performed in one of a state where the sealed chamber is rotated and a state where the sealed chamber stands still, or in a combination of these states.

5. The method for producing induced pluripotent stem cells according to any one of claims 1 to 4, wherein an internal space of the sealed chamber as defined by a side wall surface surrounding the internal space has a cylindrical shape, a geometrical rotation center line of the cylindrical shape corresponds to a center axis line of rotation movement in centrifugation in the sealed chamber,
the cylindrical shape has a bottom surface area of 90 to 200 cm², and
a relative centrifugal force acting on a content in the sealed chamber in the centrifugation is 100 to 500 G.

6. The method for producing induced pluripotent stem cells according to any one of claims 1 to 5, wherein the sealed chamber has a volume of 300 to 700 ml.

7. The method for producing induced pluripotent stem cells according to any one of claims 1 to 6, wherein the centrifugation in the step (s1) is density gradient centrifugation performed by adding a density gradient centrifugation medium into the sealed chamber.

8. The method for producing induced pluripotent stem cells according to any one of claims 1 to 7, wherein at least an inner wall surface of the sealed chamber includes a polycarbonate resin portion.

9. The method for producing induced pluripotent stem cells according to any one of claims 1 to 8, wherein the steps (s2) and (s3) are performed in the presence of a scaffold material.

10. The method for producing induced pluripotent stem cells according to claim 9, wherein the step (s1) is performed in the presence of the scaffold material.

11. The method for producing induced pluripotent stem cells according to claim 9 or 10, wherein the scaffold material is adhered to the inner wall surface of the sealed chamber.

12. The method for producing induced pluripotent stem cells according to any one of claims 9 to 11, wherein the scaffold material contains a laminin fragment, a cell-adhesive peptide and a polyvinyl acetal resin, or a cell-adhesive peptide and a poly(meth)acrylate resin.

13. The method for producing induced pluripotent stem cells according to claim 12, wherein the cell-adhesive peptide contains Arg-Gly-Asp.

14. The method for producing induced pluripotent stem cells according to any one of claims 1 to 13, further comprising, after the step (s3), a step (s4) of carrying out expansion culture of the induced pluripotent stem cells in the sealed chamber while maintaining closedness of the closed system portion.

15. A method for producing differentiated cells,
the production method comprising:
the steps (s1) to (s3) in the method for producing induced pluripotent stem cells according to any one of claims 1 to 13, and
further comprising, after the step (s3), a step (s5) of inducing differentiation of the induced pluripotent stem cells present in the sealed chamber while maintaining closedness of the closed system portion,
wherein, in the step (s5), a material necessary for the differentiation induction is supplied into the sealed chamber, and the step (s5) is performed in the sealed chamber.

16. The method according to claim 15, further comprising, between the steps (s3) and (s5), a step (s4) of carrying out expansion culture of the induced pluripotent stem cells in the sealed chamber while maintaining closedness of the closed system portion.
